# EUROPEAN PATENT APPLICATION

(11) **EP 2 371 367 A1**
(43) Date of publication of application: **05.10.2011**
(21) Application number: 10192969.3
(22) Date of filing: 21.03.2006
(51) Int. Cl.: A61K 31/568, A61K 31/58, A61P 5/28, A61P 15/00, A61P 25/16, A61P 25/28, A61K 45/06

(54) **Method of treating men with testosterone supplement and 5alpha reductase inhibitor**

(30) Priority: 25.03.2005 US 665174 P
(62) Divisional of application: 06739115.1
(71) Applicant: MERCK SHARP & DOHME (I.A.) CORP., Rahway, New Jersey 07065 (US)
(72) Inventor: Meehan, Alan, Rahway, NJ 07065-0907 (US)
(74) Representative: Buchan, Gavin MacNicol

(57) **Abstract**

A method of treating Alzheimer's disease, Parkinson's disease, sexual dysfunction or erectile dysfunction in a man by administration of a 5alpha reductase inhibitor together with a testosterone supplement is described. The method is also concerned with the use of the 5alpha reductase inhibiting compound and the testosterone supplement together with another agent useful for treating erectile dysfunction, including PDE V inhibitors; AGE (advanced glycation end-product) breakers; alpha 1 blockers; alpha 1A antagonists; alpha 2 antagonists; dopamine agonists; dopamine D4 agonists; melanocortin agonists; oxytocin agonists; prostaglandin; radical scavengers; rotamase inhibitors; aviptadil; nitroglycerine; and GPCR agonists for treating male sexual dysfunction or erectile dysfunction.

## Description

### BACKGROUND OF THE INVENTION

Alzheimer's disease (AD) is the most prevalent form of dementia. Although primarily a disease of the elderly, affecting up to 10% of the population over the age of 65, AD also affects significant numbers of younger patients with a genetic predisposition. It is a neurodegenerative disorder, clinically characterized by progressive loss of memory and cognitive function, and pathologically characterized by the deposition of extracellular proteinaceous plaques in the cortical and associative brain regions of sufferers. These plaques mainly comprise fibrillar aggregates of β-amyloid peptide (Aβ). At present there are palliative treatments, but no means to restore function in Alzheimer's patients.

Parkinson's disease (PD), is a disorder of middle or late life, with very gradual progression and a prolonged course. The most regularly observed changes in patients with PD have been in the aggregates of melanin-containing nerve cells in the brainstem (substantia nigra, locus 20 coeruleus), where there are varying degrees of nerve cell loss with reactive gliosis (most pronounced in the substantia nigra) along with distinctive eosinophilic intracytoplasmic inclusions. In its fully developed form, PD is easily recognized in patients, where stooped posture, stiffness and slowness of movement, fixity of facial expression, rhythmic tremor of the limbs, which subsides on active willed movement or complete relaxation, are common features. Generally, accompanying the other characteristics of the fully developed disorder is the festinating gait, whereby the patient, progresses or walks with quick shuffling steps at an accelerating pace as if to catch up with the body's center of gravity. The treatment of Parkinson's disease pharmacologically with levodopa combined with stereotactic surgery has only represented a partial cure, at best. Ultimately, a point seems to be reached where pharmacology can no longer compensate for the loss of basal ganglia dopamine. In addition to these motor symptoms, many patients with PD suffer from nonmotor symptoms including depression, anxiety, sexual dysfunction, decreased energy level, and an overall decline in quality of life. In a small sample of patients with PD having low T levels, Okun et al. observed that some of the refractory nonmotor symptoms of PD responded to testosterone replacement therapy [Arch. Neurol., 59:807-811(2002)].

The ability of testosterone to induce neuroprotection was reported by J. Hammond, et al., "Testosterone-mediated neuroprotection through the androgen receptor in human primary neurons," J. Neurochem., 77: 1319-1326 (2001). Gouras et al. reported that testosterone reduces secretion of Alzheimer's β-amyloid peptides and can therefore be used in the treatment of AD [(Proc. Nat. Acad. Sci., 97: 1202-1205 (2000)). In the results of a prospective longitudinal study, Moffat et al. reported that free testosterone concentrations were lower in men who developed AD, and this difference occurred before diagnosis. [Neurology 62(2):188-193(2004)].

Erectile dysfunction denotes the medical condition of inability to achieve penile erection sufficient for successful sexual intercourse. The term "impotence" is oftentimes employed to describe this prevalent condition. Erectile dysfunction can arise from either organic or psychogenic causes, with about 20% of such cases being purely psychogenic in origin. Erectile dysfunction increases from 40% at age 40, to 67% at age 75, with over 75% occurring in men over the age of 50. Until recently, only a small number of patients have received treatment because then-existing treatment alternatives, such as injection therapies, penile prosthesis implantation, and vacuum pumps, have been uniformly disagreeable. Only more recently have more viable treatment modalities become available, in particular orally active agents, such as sildenafil citrate, marketed under the brand name of VIAGRA. Sildenafil is a selective inhibitor of type V phosphodiesterase (PDE-V), a cyclic-GMP-specific phosphodiesterase isozyme. Prior to the introduction of VIAGRA on the market, less than 10% of patients suffering from erectile dysfunction received treatment. Also commercially available are CIALIS and LEVITRA.

Testosterone is converted to the more potent derivative dihydrotestosterone by the enzyme 5α-reductase. There are two isozymes of 5α-reductase in humans. One isozyme (type 1) predominates in the viscera and in the sebaceous glands of skin tissue. The other (type 2) predominates in the prostate.

Finasteride (17β-(N-tert-butylcarbamoyl)-3-oxo-4-aza-5α-androst-1-en-3-one), as shown below, is a potent inhibitor of the human type 2 enzyme. 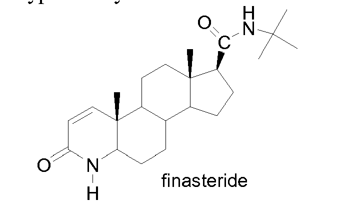 Under the tradename PROSCAR®, finasteride is known to be useful in the treatment of hyperandrogenic conditions, see e.g., U.S. 4,760,071. Finasteride is currently prescribed for the treatment of benign prostatic hyperplasia (BPH), a condition affecting to some degree the majority of men over age 55. Under the tradename PROPECIA®, finasteride is also prescribed for the treatment of male pattern hair loss.

Also known are compounds which are potent inhibitors of both 5α-reductase type 1 and type 2. These include the compound described in U.S. 5,565,467, dutasteride, sold under the tradename AVOLVE: 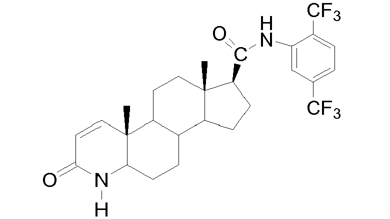 U.S. 5,719,158 ; 5,739,137; 5,910,497; and 6,001,844 and WO 97/10217 and WO 99/22728 disclose additional 5alpha-reductase inhibitors.

### BRIEF DESCRIPTION OF DRAWINGS

FIGURE 1 shows baseline and on-treatment total serum testosterone levels in a 43 year old patient treated with a topical cream (10 g) containing testosterone (T) alone (1%; 100 mg) or testosterone 1% plus finasteride (Fin; 50 mg) as measured in EXAMPLE 3.
FIGURE 2 shows baseline and on-treatment total serum dihydrotestosterone levels in a 43 year old patient treated with a topical cream (10 g) containing testosterone (T) alone (1%; 100 mg) or testosterone 1% plus finasteride (Fin, 50 mg) as measured in EXAMPLE 3.

### SUMMARY OF THE INVENTION

This invention is concerned with treating a male subject with Alzheimer's disease, Parkinson's disease, or sexual dysfunction, in particular erectile dysfunction, by administering a testosterone supplement and a 5alpha-reductase inhibiting compound. The 5alpha-reductase inhibitor and the testosterone supplement may be administered separately, sequentially or in a combined administration. In one embodiment, the 5alpha-reductase inhibiting compound is selected from one of structural formula I, II, III, and IV. This minimizes unpleasant and potentially dangerous side effects associated with administration of testosterone alone. Pharmaceutical compositions comprising a testosterone supplement and a 5alpha-reductase inhibiting compound are another aspect of the present invention.

The use of the 5-alpha reductase inhibiting compound and testosterone supplement together with other agents useful for treating Alzheimer's disease, including: tacrine, rivastigmine, galantamine, memantine, antioxidants (vitamins E and C, selenium), Ginkgo biloba, short or medium acting benzodiazepines, cholinergic enhancing agents (donepezil, leuprolide acetate), and nonsteroidal antiinflammatory drugs is also described. The 5alpha-reductase compound, testosterone supplement and other agent useful for treating Alzheimer's disease may be administered separately, sequentially or in a combined preparation.

The use of the 5-alpha reductase inhibiting compound and testosterone supplement together with other agents useful for Parkinson's disease, including: levodopa/carbidopa, levodopa/benserazide, dopamine receptor agonists (eg., ropinirole, apomorphine, selegiline, entacapone, bromocryptine, carbergoline, lysuride, pergolide, antimuscarinic drugs (eg., orphenadrine, bezhexol, benztropine and procyclidine), ethopropazine, trihexphenidyl, antidepressants (amitryptaline, doxepine, imipramine, nortriptyline, propanolol), antihistamines (diphenhydramine, orphenadrine), and amantadine is also described. The 5alpha-reductase compound, testosterone supplement and other agent useful for treating Parkinson's disease may be administered separately, sequentially or in a combined preparation.

The use of the 5-alpha reductase inhibiting compound and testosterone supplement together with other agents useful for treating male sexual dysfunction, including: PDE V inhibitors; AGE (advanced glycation end-product) breakers; alpha 1 blockers; alpha 1A antagonists; alpha 2 antagonists; dopamine agonists; dopamine D4 agonists; melanocortin agonists; oxytocin agonists; prostaglandins; radical scavengers; arotamase inhibitors; aviptadil; nitroglycerine; and GPCR agonists is also described. The 5alpha-reductase compound, testosterone supplement and other agent useful for treating male sexual dysfunction may be administered separately, sequentially or in a combined preparation.

### DETAILED DESCRIPTION OF THE INVENTION

The present invention is directed to a method of treating a male subject with Alzheimer's disease, Parkinson's disease, sexual dysfunction, or erectile dysfunction comprising combined administration of a 5 alpha-reductase inhibitor and a testosterone supplement. The 5alpha-reductase inhibitor and the testosterone supplement may be administered separately, sequentially or in a combined administration. One embodiment is directed to a method of treating a male subject with Parkinson's disease comprising combined administration of a 5alpha reductase type 2 inhibiting compound or a dual 5 alpha reductase type 1/type 2 inhibiting compound or a 5alpha reductase type 2 inhibiting compound together with a 5 alpha reductase 1 inhibiting compound, together with a testosterone supplement. Another embodiment is directed to a method of treating a male subject with Alzheimer's disease comprising combined administration of a 5alpha reductase type 2 inhibiting compound or a dual 5 alpha reductase type 1/type 2 inhibiting compound or a 5alpha reductase type 2 inhibiting compound together with a 5 alpha reductase 1 inhibiting compound, together with a testosterone supplement. Yet another embodiment is directed to a method of treating a male subject with sexual dysfunction or erectile dysfunction comprising combined administration of a 5alpha reductase type 2 inhibiting compound or a dual 5 alpha reductase type 1/type 2 inhibiting compound or a 5alpha reductase type 2 inhibiting compound together with a 5 alpha reductase 1 inhibiting compound, together with a testosterone supplement.

Aging men with Alzheimer's disease, Parkinson's disease, sexual dysfunction or erectile dysfunction often also have low T levels. It has been suggested that the low T levels may contribute to the symptomatology and/or the development and progression of these diseases in these men. The present invention solves the problem of safely elevating T levels in aging men using a well tolerated, pharmacologic therapy that does not elevate DHT levels. In particular, given the specific distribution of 5α-reductase in the CNS and periphery, the concomitant administration of T and a 5α-reductase inhibitor allows for localized elevation of T in target organs/tissues of interest (eg, the brain). Previously, there were no pharmacologic therapies available that could safely correct the low/low-normal T levels in these men without also significantly increasing DHT levels and/or increasing the levels of the potentially mitogenic gonadotrophins. In particular, the absence of 5alpha-reductase inhibition, treatment of middle aged and agining men with a T supplement results in a significant elevation in DHT levels (up to 4-fold), this increase in DHT levels and accompanying risks associated with DHT levels, can be reversed by concomitant therapy with a 5alpha-reductase inhibitor.

Men with Alzheimer's disease or Parkinson's disease are often being treated for depression, and the current trend is to employ non-androgen modulating antidepressants for the treatment of this depression. The use of non-androgen modulating antidepressants can mask partial androgen deficiency that may be contributing significantly to the overall depression typically experienced by aging men with Alzheimer's disease and Parkinson's disease. If left untreated, the partial androgen deficiency may lead to a significant increase in insulin resistance, visceral adiposity, and hypertriglyceridemia, thereby enhancing overall cardiovascular risk.

In one embodiment, the testosterone supplement is selected from: testosterone, testosterone precursors, prodrugs, analogs, and other androgen receptor agonists such as dehydroepiandrosterone, androstenedione, testosterone enanthate, testoterone propionate, testosterone cypionate, testosterone undecanoate, testosterone cyclodextrin, methyltestosterone, fluoxy mesterone, 17α-methyl testosterone, ANDROGEL-DHT gel; Balasterone (7alpha, 17beta)- 17 hydroxy-7, 17-dimethylandrost-4-en-3-on3 (MYAGEN); clostebol (17beta)-4-chloro-17-hydroxyandrost-4-en-3-one; formebolone (11alpha, 17beta)-dihydroxy-17-methyl-3-oxo-androsta-1,4-diene-2-caroxaldehyde (ESICLENE); Nadrolone (17beta)-17-hydroxyestr-4-en-3-one (NORLONGANDRON, NOR-DURANDRON, SANABOLICUM, DECA-DURBOLINE, DECA-DURABOL, DECAHYBOLIN, HYBOLIN DECANOATE, RETABOLIL, LAURABOLIN, DEMELON, ANADOR, ANADUR, ACTIVIN, DURABOL, STRABOLENE, SUPERANABOLON, NADROLIN, NORYBOL-19, NORTESTO); oxymesterone (17beta)-4,17-dihydroxy-17methylandrost-4-en-3 -one; quinbolone (17beta)- 17-(1-cyclopenten-1-yloxyandrosta-1,4-dien-3-one (ANABOLICUM VISTA); and other androgen receptor agonists, including salts and ester derivatives thereof.

In one class of this embodiment, the testosterone supplement is selected from: testosterone, testosterone enanthate, testoterone propionate, testosterone cypionate, testosterone undecanoate, testosterone cyclodextrin, methyltestosterone, fluoxy mesterone, and 17-α methyl testosterone.

In another class of this embodiment, the testosterone supplement is selected from testosterone and testosterone ester derivatives.

One embodiment is directed to a method of treating a male subject with Alzheimer's disease, Parkinson's disease, sexual dysfunction, or erectile dysfunction by administration of a 5alpha reductase inhibiting compound of structural formula I, II, III or IV: 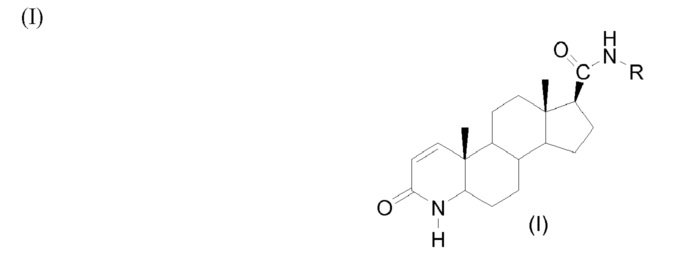 wherein R is selected from:
(a) C₁₋₁₀ alkyl, unsubstituted or substituted with one to three halogen substituents, and
(b) phenyl, unsubstituted or substituted with one to three substituents independently selected
   from halogen, methyl, and trifluoromethyl; 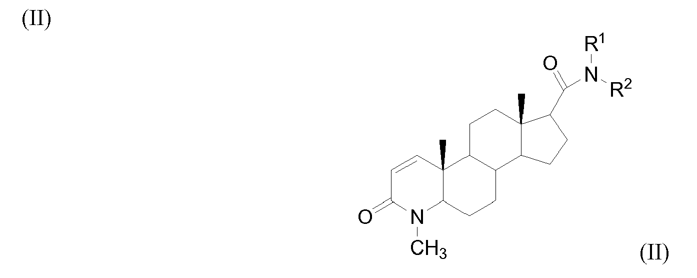 wherein:
   R¹ is selected from
      (a) H, and
      (b) C₁₋₆ alkyl;
   R² is selected from:
      (a) diarylmethyl, either unsubstituted or substituted on one or both of the aryl rings with one to three substituents independently selected from:
         (1) halo (F, Cl, Br, I),
         (2) C₁₋₂ alkyl,
         (3) trifluoromethyl,
         (4) nitro,
         (5) hydroxy,
         (6) cyano,
         (7) phenyl,
         (8) C₁₋₂ alkyloxy,
         (9) heteroaryl,
         (10) S(O)ₙR³, wherein n is selected from 0, 1, and 2, and
         (11) alkyoxy;
      (b) phenyl substituted with one to three substituents independently selected from:
         (1) halo (F, Cl, Br, I),
         (2) C₁₋₂ alkyl;
         (3) trifluoromethyl,
         (4) nitro,
         (5) hydroxy,
         (6) cyano,
         (7) phenyl,
         (8) C₁₋₂ alkyloxy,
         (9) heteroaryl,
         (10) S(O)ₙR³, wherein n is selected from 0, 1, and 2, and
         (11) alkyoxy;
      (c) heteroaryl, either unsubstituted or substituted with one to three substituents independently selected from:
         (1) halo (F, Cl, Br, I),
         (2) C₁₋₂ alkyl;
         (3) trifluoromethyl,
         (4) nitro,
         (5) hydroxy,
         (6) cyano,
         (7) amino,
         (8) C₁₋₂ alkyloxy,
         (9) phenyl, and
         (10) heteroaryl;
   R³ is selected from:
      (a) C₁₋₄ alkyl,
      (b) phenyl, and
      (c) heteroaryl; 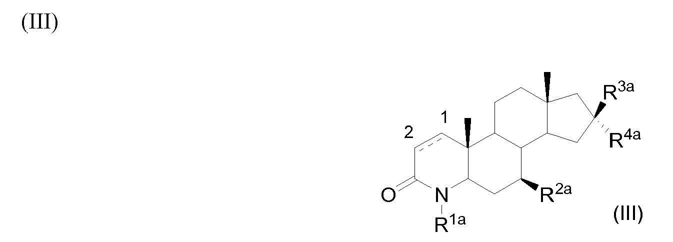 wherein:
         the C1-C2 carbon-carbon bond may be a single bond, or a double bond as indicated by the dashed line;
         R^{1a} is selected from the group consisting of hydrogen and methyl;
         R^{2a} is selected from the group consisting of hydrogen and C₁₋₁₀ alkyl;
         one of R^{3a} and R^{4a} is selected from the group consisting of hydrogen and methyl, and the other is selected from the group consisting of:
         (a) amino;
         (b) cyano;
         (c) fluoro,
         (d) methyl;
         (e) OH;
         (f) -C(O)NR_{b}R_{c}, where R_{b} and R_{c} are independently H, C₁₋₆ alkyl, aryl, or arylC_{1- 6}alkyl; wherein the alkyl moiety can be substituted with 1-3 of: halo; C₁₋₄alkoxy; or trifluoromethyl; and the aryl moiety can be substituted with 1-3 of: halo; C₁₋₄alkyl; C₁₋₄ alkoxy; or trifluoromethyl;
         (g) C₁₋₁₀ alkyl-X-;
         (h) C₂₋₁₀ alkenyl-X-; wherein the C₁₋₁₀ alkyl in (g) and C₂₋₁₀alkenyl in (h) can be unsubstituted or substituted with one to three of:
            (i) halo; hydroxy; cyano; nitro; mono-, di- or trihalomethyl; oxo; hydroxysulfonyl; carboxy;
            *(ii)* hydroxyC₁₋₆alkyl; C₁₋₆alkyloxy; C₁₋₆ alkylthio; C₁₋₆alkylsulfonyl; C_{1- 6} alkyloxycarbonyl; in which the C₁₋₆ alkyl moiety can be further substituted with 1-3 of: halo; C₁₋₄ alkoxy; or trifluoromethyl;
            *(iii)* arylthio; aryl; aryloxy; arylsulfonyl; aryloxycarbonyl; in which the aryl moiety can be further substituted with 1-3 of: halo; C₁₋₄ alkyl; C₁₋₄ alkoxy; or trifluoromethyl;
            *(iv)* -C(O)NR_{b}R_{c}; -N(R_{b})-C(O)-R_{c}; -NR_{b}R_{c}; where R_{b} and R_{c} are defined above;
         (i) aryl-X-;
         (j) heteroaryl-X-, wherein heteroaryl is a 5, 6 or 7 membered heteroaromatic ring containing at least one member selected from the group consisting of: one ring oxygen atom, one ring sulfur atom, 1-4 ring nitrogen atoms , or combinations thereof; in which the heteroaromatic ring can also be fused with one benzo or heteroaromatic ring; wherein the aryl in (i) and heteroaryl in (j) can be unsubstituted or substituted with one to three of:
            (v) halo; hydroxy; cyano; nitro; mono-, di- or trihalomethyl; mono-, di- or trihalomethoxy; C₂₋₆ alkenyl; C₃₋₆ cycloalkyl; formyl; hydrosulfonyl; carboxy; ureido;
            (vi) C₁₋₆ alkyl; hydroxy C₁₋₆ alkyl; C₁₋₆ alkyloxy; C₁₋₆ alkyloxy C₁₋₆alkyl; C₁₋₆ alkylcarbonyl; C₁₋₆ alkylsulfonyl; C₁₋₆ alkylthio; C₁₋₆ alkylsulfinyl; C₁₋₆ alkylsulfonamido; C₁₋₆ alkylarylsulfonamido; C₁₋₆ alkyloxy-carbonyl; C₁₋₆ alkyloxycarbonyl C₁₋₆alkyl; R_{b}R_{c}N-C(O)-C₁-₆alkyl; C₁₋₆ alkanoylamino C₁₋₆ alkyl; aroylamino C₁₋₆ alkyl; wherein the C₁₋₆ alkyl moiety can be substituted with 1-3 of: halo; C₁₋₄alkoxy; or trifluoromethyl;
            *(vii)* aryl; aryloxy; arylcarbonyl; arylthio; arylsulfonyl; arylsulfinyl; arylsulfonamido; aryloxycarbonyl; wherein the aryl moiety can be substituted with 1-3 of: halo; C₁₋₄alkyl; C₁₋₄alkoxy; or trifluoromethyl;
            *(viii)* -C(O)NR_{b}R_{c}; -O-C(O)-NR_{b}R_{c}; -N(Rb)-C(O)-R_{c}; -NR_{b}R_{c}; Rb-C(O)-N(R_{c})-; where R_{b} and R_{c} are defined in (f) above; and -N(R_{b})-C(O)-OR_{g} wherein Rg is C₁₋₆alkyl or aryl, in which the alkyl moiety can be substituted with 1-3 of: halo; C₁₋₄alkoxy; or trifluoromethyl, and the aryl moiety can be substituted with 1-3 of: halo; C₁₋₄alkyl; C₁₋₄ alkoxy, or trifluoromethyl; -N(R_{b})-C(O) NR_{c}R_{d}, wherein R_{d} is selected from H, C₁₋₆ alkyl, and aryl; in which said C₁₋₆alkyl and aryl can be substituted as described above in (f) for R_{b} and R_{c};
            (ix) a heterocyclic group, which is a 5, 6 or 7 membered ring, containing at least one member selected from the group consisting of: one ring oxygen atom, one ring sulfur atom, 1-4 ring nitrogen atoms, or combinations thereof; in which the heterocyclic ring can be aromatic, unsaturated, or saturated, wherein the heterocyclic ring can be fused with a benzo ring, and
               wherein said heterocyclic ring can be substituted with one to three substituents, as defined above for v), *vi*), *vii*) and *viii*), excluding ix) a heterocyclic group; and
         (k) R^{3a} and R^{4a} taken together can be carbonyl oxygen;
         (1) R^{3a} and R^{4a} taken together can be =CH-Rg wherein Rg is defined in *viii*); and wherein:
            X is selected from the group consisting of:
               - O-; -S(O)ₙ-; -C(O)-; -CH(Rₑ)-; -C(O)-O-*; -C(O)-N(Rₑ)-*;
               - N(Rₑ)-C(O)-O-*; -O-C(O)-N(Rₑ)-*; -N(Rₑ)C(O)-N(Rₑ)-;
               - O-CH(Rₑ)-*; -N(Re)-; wherein Rₑ is H, C₁₋₃ alkyl, aryl, aryl- C₁₋₃ alkyl, or unsubstituted or substituted heteroaryl, as defined above in (j); wherein the asterisk (*) denotes the bond which is attached to the 16-position in Structure III; and n is zero, 1 or 2;
                  and wherein each alkyl and alkenyl moiety can be unsubstituted or substituted with one or more, and preferably 1 to three, of:
                  (*i*) halo; hydroxy; cyano; nitro; mono-, di- or trihalomethyl; oxo; hydroxysulfonyl; carboxy;
                  *(ii)* hydroxyC ₁₋₆alkyl; C ₁₋₆alkyloxy; C ₁₋₆ alkylthio; C ₁₋₆alkylsulfonyl; C ₁₋₆ allcyloxycarbonyl; in which the C₁₋₆ alkyl moiety can be further substituted with 1-3 of: halo; C₁₋₄ alkoxy; or trifluoromethyl;
                  *(iii)* arylthio; aryl; aryloxy; arylsulfonyl; aryloxycarbonyl; in which the aryl moiety can be further substituted with 1-3 of: halo; C₁₋₄ alkyl; C₁₋₄ alkoxy; or trifluoromethyl; and
                  (iv) -C(O)NR_{b}R_{c}; -N(R_{b})-C(O)-R_{c}; -NR_{b}R_{c}; where R_{b} and R_{c} are defined above; and halo is F, Cl, Br or I;
                     or 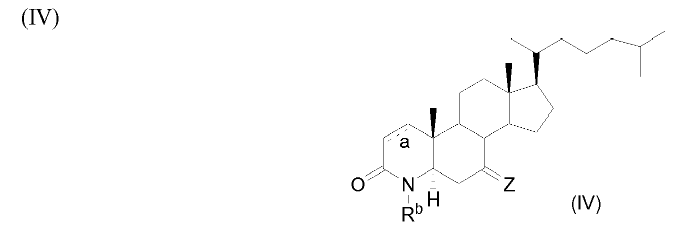
                  wherein:
                  R^{b} is selected from hydrogen and methyl;
                     the dashed line " - - - " a represents a single bond or a double bond;
                  =Z is selected from:
                     (1) oxo,
                     (2) α-hydrogen and a β-substituent selected from:
                        (a) C₁-C₄ alkyl,
                        (b) C₂-C₄ alkenyl,
                        (c) CH₂COOH,
                        (d) -OH,
                        (e) -COOH,
                        (f) -COO(C₁-C4 alkyl),
                        (g) -OCONR^{1b}R^{2b} wherein R^{1b} and R^{2b} independently are selected from:
                           (i) H,
                           (ii) C₁-C₄ alkyl,
                           (iii) phenyl, and
                           (iv) benzyl, or
                              R^{1b} and R^{2b} together with the nitrogen atom to which they are attached represent
                              a 5-6 membered saturated heterocycle, optionally containing one other heteratom selected from -O-, -S- and -N(R')- wherein R' is -H or methyl;

                     (h) C₁-C₄ alkoxy,
                     (i) C₃-C₆ cycloalkoxy,
                     (j) -OC(O)-C₁₋₄ alkyl,
                     (k) halo,
                     (1) hydroxy-C₁-C₂ alkyl,
                     (m) halo-C₁-C₂ alkyl,
                     (n) -CF₃ and
                     (o) C₃-C₆ cycloalkyl;
                        (3) =CHR^{3b}; wherein R^{3b} is selected from -H and C₁-C₄ alkyl;
            or a pharmaceutically acceptable salt thereof;
            together with a testosterone supplement.

Combinations of substituents and/or variables are permissible only if such combinations result in stable compounds.

As used herein "alkyl" is intended to include both branched- and straight-chain saturated aliphatic hydrocarbon groups having the specified number of carbon atoms, e.g., methyl (Me), ethyl (Et), propyl, butyl, pentyl, hexyl, heptyl, octyl, nonyl, decyl, iso-propyl (i-Pr), iso-butyl (i-Bu), tert-butyl (t-Bu), secbutyl (s-Bu), iso-pentyl, and the like. "Alkyloxy" (or "alkoxy") represents an alkyl group having the indicated number of carbon atoms attached through an oxygen bridge, e.g., methoxy, ethoxy, propyloxy, and the like. "Alkenyl" is intended to include hydrocarbon groups of either a straight or branched configuration with one or more carbon-carbon double bonds which may occur in any stable point along the chain, such as ethenyl, propenyl or allyl, butenyl, pentenyl, and the like. Included in this invention are all E, Z diastereomers.

The term "C₃-C₆ cycloalkyl" as used herein is meant to include cyclopropyl, cyclobutyl, cyclopentyl, and cyclohexyl.

The term "halo" and/or "halogen" as used herein is meant to include fluoro, chloro, bromo, and iodo.

The term "oxo", as used herein, indicates an oxo radical which can occur in any stable point along the carbon chain resulting in a formyl group, if at the end of the chain, or an acyl or aroyl group at other points along the carbon chain.

As used herein the term "aryl", i.e., C₆₋₁₀ aryl, is intended to mean phenyl or naphthyl, including 1-naphthyl or 2-naphthyl, either unsubstituted or substituted as described below.

The term "heteroaryl" as used herein, is intended to include a 5, 6 or 7 membered heteroaromatic radical containing at least one member selected from the group consisting of: one ring oxygen atom, one ring sulfur atom, 1-4 ring nitrogen atoms, or combinations thereof; in which the heteroaryl ring can also be fused with one benzo or heteroaromatic ring. This category includes the following either unsubstituted or substituted heteroaromatic rings (as described below): pyridyl, furyl, pyrryl, thienyl, isothiazolyl, imidazolyl, benzimidazolyl, tetrazolyl, pyrazinyl, pyrimidyl, quinolyl, quinazolinyl, isoquinolyl, benzofuryl, isobenzofuryl, benzothienyl, pyrazolyl, indolyl, isoindolyl, purinyl, carbazolyl, isoxazolyl, thiazolyl, isothiazolyl, oxazolyl, benzthiazolyl, and benzoxazolyl. In one embodiment, heteroaryl is selected from: pyridyl, pyrazinyl, pyrazolyl and thiazolyl. The heteroaryl ring may be attached by a nitrogen, or carbon atom in the ring, which results in the creation of a stable structure. The heteroaryl ring can also be fused to a benzo ring.

The fused heteroaromatic ring systems include: purine, imidazoimidazole, imidazothiazole, pyridopyrimidine, pyridopyridazine, pyrimidopyrimidine, imidazopyridazine, pyrrolopyridine, imidazopyridine, and the like.

The "heterocyclic" group includes the fully unsaturated heteroaryl rings described above and also their respective dihydro, tetrahydro and hexahydro derivatives resulting in partially unsaturated and fully saturated versions of the ring systems. Examples include: dihydroimidazolyl, dihydrooxazolyl, dihydropyridyl, tetrahydrofuryl, dihydropyrryl, tetrahydrothienyl, dihydroisothiazolyl, 1,2-dihydrobenz-imidazolyl, 1,2-dihydrotetrazolyl, 1,2-dihydropyrazinyl, 1,2-dihydro-pyrimidyl, 1,2-dihydroquinolyl, 1,2,3,4-tetrahydroisoquinolyl, 1,2,3,4-tetrahydrobenzofuryl, 1,2,3,4-tetrahydroisobenzofuryl, 1,2,3,4-tetra-hydrobenzothienyl, 1,2,3,4-tetrahydropyrazolyl, 1,2,3,4-tetrahydro-indolyl, 1,2,3,4-tetrahydroisoindolyl, 1,2,3,4-tetrahydropurinyl, 1,2,3,4-tetrahydrocarbazolyl, 1,2,3,4-tetrahydroisoxazolyl, 1,2,3,4-tetrahydro-thiazolyl, 1,2,3,4-tetrahydrooxazolyl, 1,2,3,4-tetrahydrobenzthiazolyl, and 1,2,3,4-tetrahydrobenzoxazolyl. and the like.

The heterocyclic group can be substituted in the same fashion as described above for heteroaryl.

Whenever the terms "alkyl", "alkenyl", "alkyloxy (or alkoxy)", "aryl" or "heteroaryl", or one of their prefix roots, appear in a name of a substituent, (e.g., aralkoxyaryloxy) they shall have the same definitions as those described above for "alkyl", "alkenyl", "alkyloxy (or alkoxy)", "aryl" and "heteroaryl", respectively. Designated numbers of carbon atoms (e.g., C₁₋₁₀) shall refer independently to the number of carbon atoms in an alkyl or alkenyl moiety or to the alkyl or alkenyl portion of a larger substituent in which alkyl or alkenyl appears as its prefix root.

Many organic compounds can form complexes with solvents in which they are reacted or from which they are precipitated or crystallized. These complexes are known as "solvates". Solvates of compounds of structural formula I, II, II and IV are within the scope of the present invention. Many organic compounds can exist in more than one crystalline form. For example, crystalline form may vary from solvate to solvate. Thus, all crystalline forms of the compounds of structural formula I or the pharmaceutically acceptable solvates thereof are within the scope of the present invention.

One aspect provides a method for the treatment or prevention of Alzheimer's disease, Parkinson's disease, or sexual dysfunction, including erectile dysfunction, in a man which comprises administering to a patient in need of such treatment or prevention a therapeutically or prophylactically effective amount of a 5alpha-reductase inhibitor and a testosterone supplement.

Another aspect provides a method for the treatment or prevention of Parkinson's disease in a man which comprises administering to a patient in need of such treatment or prevention a therapeutically or prophylactically effective amount of a 5alpha-reductase inhibitor and a testosterone supplement.

Another aspect provides a method for the treatment or prevention of Alzheimer's disease in a man which comprises administering to a patient in need of such treatment or prevention a therapeutically or prophylactically effective amount of a 5alpha-reductase inhibitor and a testosterone supplement.

Yet another aspect provides a method for the treatment or prevention of male sexual dysfunction including erectile dysfunction in a man which comprises administering to a patient in need of such treatment or prevention a therapeutically or prophylactically effective amount of a 5alpha-reductase inhibitor and a testosterone supplement.

Another aspect provides a pharmaceutical composition comprising a 5alpha-reductase inhibitor and a testosterone supplement for separate, sequential or simultaneous administration.

Yet another aspect provides a method for the treatment or prevention of Alzheimer's disease, Parkinson's disease or male sexual dysfunction, including erectile dysfunction in a man, which comprises administering to a male patient in need of such treatment or prevention a therapeutically or prophylactically effective amount of a 5alpha-reductase inhibitor and a testosterone supplement in combination with a therapeutically effective amount of another agent known to be useful for the treatment of these conditions.

Still another aspect provides a method for treating a male subject with Alzheimer's disease, Parkinson's disease or sexual dysfunction comprising administration to the subject of a therapeutically effective amount of a testosterone supplement together with a 5alpha-reductase inhibitor, selected from: a 5alpha-reductase type 2 inhibitor, a dual 5alpha-reductase type 1/type 2 inhibitor, and a 5alpha-reductase type 2 inhibitor and a 5alpha-reductase type 1 inhibitor.

Yet another aspect provides a method for the treatment or prevention of a condition selected from:
Alzheimer's disease, Parkinson's disease, and male sexual dysfunction, including erectile dysfunction, in a man which comprises administering to a patient in need of such treatment or prevention a therapeutically or
prophylactically effective amount of a 5alpha reductase inhibiting compound of structural formula I, II, III or IV and a testosterone supplement in combination with a therapeutically effective amount of another agent known to be useful for the treatment of the condition.

One embodiment comprises administration of a compound of structural formula I with a testosterone supplement.

In one class of this embodiment, R is selected from:
(a) unsubstituted C₁₋₁₀ alkyl, and
(b) phenyl unsubstituted or substituted with one or two trifluoromethyl substituents.
   In a subclass of this class of compounds of structural formula I, R is t-butyl.

In another subclass of this class of structural formula I, R is 2,5-bis(trifluoromethyl)phenyl.

Another embodiment comprises administration of a compound of structure Formula II with a testosterone supplement. In one class of this embodiment, R² is diarylmethyl, either unsubstituted or substituted on an aryl moiety with one to three substituents independently selected from:
(1) halo (F, Cl, Br, I),
(2) C₁₋₂ alkyl,
(3) trifluoromethyl,
(4) nitro,
(5) hydroxy,
(6) cyano,
(7) phenyl,
(8) C₁₋₂ alkyloxy,
(9) heteroaryl,
(10) S(O)ₙR³, wherein n is selected from 0, 1, and 2, and
(11) alkyoxy.

In one subclass of this class are compounds wherein R² is unsubstituted diphenylmethyl.

Examples of compounds of structural formula II of this subclass include: *N*-(diphenylmethyl)-4-methyl-3-oxo-4-aza-5α-androst-1-ene-17β-carboxamide; *N*-(diphenylmethyl)-N-methyl-4-methyl-3-oxo-4-aza-5α-androst-1-ene-17β-carboxamide.

In another class of the present embodiment, are compounds of Formula II wherein R² is phenyl substituted with one to three substituents independently selected from
(1) halo (F, Cl, Br, I),
(2) C₁₋₂ alkyl,
(3) trifluoromethyl,
(4) nitro,
(5) hydroxy,
(6) cyano,
(7) phenyl,
(8) C₁₋₂ alkyloxy,
(9) heteroaryl,
(10) S(O)ₙR³, wherein n is selected from 0, 1, and 2, and
(11) alkyoxy.

Examples of compounds of structural formula II of this class are:
*N*-(2-methylphenyl)-3-oxo-4-aza-4-methyl-5α-androst-1-ene-17β-carboxamide;
*N*-(2-methoxyphenyl)-3-oxo-4-aza-4-methyl-5α-androst-1-ene-17β-carboxamide;
*N*(2-chlorophenyl)-3-oxo-4-aza-4-methyl-5α-androst -1-ene-17β-carboxamide;
*N*(4-chlorophenyl)-3-oxo-4-aza-4-methyl-5α-androst -1-ene-17β-carboxamide;
*N*-(2-fluorophenyl)-3-oxo-4-aza-4-methyl-5α-androst-1-ene-17β-carboxamide;
*N*-(2-trifluoromethyl-phenyl)-3-oxo-4-aza-4-methyl-5α-androst-1-ene-17β-carboxamide;
*N*(2,5-bistrifluoromethyl-phenyl)-3-oxo-4-aza-4-methyl-5α-androst-1-ene-17β-carboxamide;
*N*-(2-biphenyl)-3-oxo-4-aza-4-methyl-5α-androst-1-ene-17β-carboxamide;
*N*-(4-biphenyl)-3-oxo-4-aza-4-methyl-5α-androst-1-ene-17β-carboxamide.

In another class of this embodiment of structural formula II, R² is heteroaryl, either unsubstituted or substituted with one to three substituents independently selected from:
(1) halo (F, Cl, Br, I),
(2) C₁₋₂ alkyl,
(3) trifluoromethyl,
(4) nitro,
(5) hydroxy,
(6) cyano,
(7) amino,
(8) C₁₋₂ alkyloxy,
(9) phenyl, and
(10) heteroaryl;

In one subclass of this embodiment, heteroaryl is pyridyl, pyrazinyl, pyrazolyl, or thiazolyl.

Examples of compounds of structural Formula II of this subclass are:
*N*-(4-pyridyl)-3-oxo-4-methyl-4-aza-5α-androst-1-ene-17β-carboxamide,
*N*-(3-pyridyl)-3-oxo-4-methyl-4-aza-5α-androst-1-ene-17β-carboxamide,
*N*-(pyrazinyl)-3-oxo-4-methyl-4-aza-5α-androst-1-ene-17β-carboxamide,
*N*-(3-pyrazoyl)-3-oxo-4-methyl-4-aza-5α-androst-1-ene-17β-carboxamide, and
*N*-(2-thiazolyl)-3-oxo-4-aza-4-methyl-5α-androst-1-ene-17β-carboxamide.

One embodiment comprises administration of a compound of structural formula III with a testosterone supplement.

In one class of this embodiment, the C₆₋₁₀ aryl and heteroaryl groups in Formula III are unsubstituted or substituted from one, two, or three substituents independently selected from:
(*v* ) halo; hydroxy; cyano; nitro; mono-, di- or trihalomethyl; mono-, di- or trihalomethoxy; C₂₋₆ alkenyl; C₃₋₆ cycloalkyl; formyl; hydrosulfonyl; carboxy; ureido;
(vi) C₁₋₆ alkyl; hydroxy C₁₋₆ alkyl; C₁₋₆ alkyloxy; C₁₋₆ alkyloxy C₁₋₆alkyl; C₁₋₆ alkylcarbonyl; C₁₋₆ alkylsulfonyl; C₁₋₆ alkylthio; C₁₋₆ alkylsulfinyl; C₁₋₆ alkylsulfonamido; C₁₋₆ alkylarylsulfonamido; C₁₋₆ alkyloxy-carbonyl; C₁₋₆ alkyloxycarbonyl C₁₋₆alkyl; R_{b}R_{c}N-C(O)-C₁-₆alkyl; C₁₋₆ alkanoylamino C₁₋₆ alkyl; aroylamino C₁₋₆ alkyl; wherein the C₁₋₆ alkyl moiety can be substituted with 1-3 of: halo; C₁₋₄alkoxy; or trifluoromethyl;
(*vii*) aryl; aryloxy; arylcarbonyl; arylthio; arylsulfonyl; arylsulfinyl; arylsulfonamido; aryloxycarbonyl; wherein the aryl moiety can be substituted with 1-3 of: halo; C₁₋₄alkyl; C₁₋₄alkoxy; or trifluoromethyl;
(*viii*) -C(O)NR_{b}R_{c}; -O-C(O)-NR_{b}R_{c}; -N(Rb)-C(O)-R_{c}; -NR_{b}R_{c}; Rb-C(O)-N(R_{c})-; where R_{b} and R_{c} are defined in (e) above; and -N(R_{b})-C(O)-OR_{c}, wherein this instance R_{c} is C₁₋₆alkyl or aryl; -N(R_{b})-C(O) NR_{c}R_{d}, wherein R_{d} is selected from H, C₁₋₆ alkyl, and aryl; in which said C₁₋₆alkyl and aryl can be substituted as described above in (e) for R_{b} and R_{c};
(ix) a heterocyclic group, which is a 5, 6 or 7 membered ring, containing at least one member selected from the group consisting of: one ring oxygen atom, one ring sulfur atom, 1-4 ring nitrogen atoms, or combinations thereof; in which the heterocyclic ring can be aromatic, unsaturated, or saturated, and wherein the heterocyclic ring can be fused with a benzo ring, and wherein said heterocyclic ring can be substituted with one to three substituents, as defined above for v), *vi*), *vii*) and *viii*), excluding ix) a heterocyclic group.

Particular compounds of structural formula III, useful in the methods of the present invention include: 4-aza-4,7β-dimethyl-5α-androstane-3,16-dione; 4-aza-4-methyl-5α-androstan-3,16-dione; 3-oxo-4-aza-4-methyl-16β-hydroxy-5α-androstane; 3-oxo-4-aza-4-methyl-16β-(benzylaminocarbonyloxy)-5α-androstane; 3-oxo-4-aza-4-methyl-16β-benzoylamino-5α-androstane; 3-oxo-4-aza-4-methyl-16β-methoxy-5α-androstane; 3-oxo-4-aza-4-methyl-16β-allyloxy-5α-androstane; 3-oxo-4-aza-4-methyl-16β-(n-propyloxy)-5α-androstane; 3-oxo-4-aza-4-methyl-16α-hydroxy-5α-androstane; 3-oxo-4-aza-4-methyl-16β-(phenoxy)-5α-androstane; 3-oxo-4-aza-7β-methyl-16β-(phenoxy)-5α-androst-1-ene; 3-oxo-4-aza-4-methyl-16α-methoxy-5α-androstane; 3-oxo-4-aza-4-methyl-16β-(4-chlorophenoxy)-5α-androstane; 3-oxo-4-aza-7β-methyl-16β-(4-chlorophenoxy)-5α-androst-1-ene; 3-oxo-4-aza-7β-methyl-16β-(4-chloro-phen-oxy)-5α-androstane; 3-oxo-4-aza-7β-methyl-16β-(3-chloro-4-methylphenoxy)-5α-androstane; 3-oxo-4-aza-7β-methyl-16β-(4-methylphenoxy)-5α-androstane; 3-oxo-4-aza-7β-methyl-16β-(4-methylphenoxy)-5α-androst-1-ene; 3-oxo-4-aza-7β-methyl-16β-[4-(1-pyrrolyl)phenoxy]-5α-androst-1-ene; 3-oxo-4-aza-4,7β-dimethyl-16p-hydroxy-5α-androstane; 3-oxo-4-aza-4,7β-dimethyl-16β-methoxy-5α-androstane; 3-oxo-4-aza-4,7β-dimethyl-16β-allyloxy-5α-androstane; 3-oxo-4-aza-4,7β-dimethyl-16β-(3,3-dimethyl-allyloxy)-5α-androstane; 3-oxo-4-aza-4,7β-dimethyl-16β-(n-propyloxy)-5α-androstane; 3-oxo-4-aza-4,7β-dimethyl-16β-(iso-pentoxy)-5α-androstane; 3-oxo-4-aza-4,16α-dimethyl-16β-hydroxy-5α-androstane; 3-oxo-4-aza-4,7β-dimethyl-16β-ethyloxy-5α-androstane; 3-oxo-4-aza-4,7β-dimethyl-16β-benzyloxy-5α-androstane; 3-oxo-4-aza-4,7β-dimethyl-16α-hydroxy-5α-androstane; 3-oxo-4-aza-4,7β-dimethyl-16β-methylthio-5α-androstane; 3-oxo-4-aza-4,7β-dimethyl-16β-(n-propylthio)-5α-androstane; 3-oxo-4-aza-4,7β-dimethyl-16β-fluoro-5α-androstane; 3-oxo-4-aza-4,7β-dimethyl-16β-cyano-5α-androstane; 3-oxo-4-aza-4,7β-dimethyl-16β-(1-hexyl)-5α-androstane; 3-oxo-4-aza-4,7β-dimethyl-16β-(n-propyl)-5α-androstane; 3-oxo-4-aza-4,7β-dimethyl-16β-benzyl-5α-androstane; 3-oxo-4-aza-4,7β-dimethyl-16β-(4-chlorobenzyl)-5α-androstane; 3-oxo-4-aza-4,16α-dimethyl-16β-methoxy-5α-androstane; 3-oxo-4-aza-4,7β-dimethyl-16β-(4-cyanophenoxy)-5α-androstane; 3-oxo-4-aza-4,7β-dimethyl-16β-(3-cyanophenoxy)-5α-androstane; 3-oxo-4-aza-4,7β-dimethyl-16β-(4-nitrophenoxy)-5α-androstane; 3-oxo-4-aza-4,7β-dimethyl-16β-(1-naphthyloxy)-5α-androstane; 3-oxo-4-aza-4,7β-dimethyl-16β-(3-chloro-4-methyl-phen-oxy)-5α-androstane; 3-oxo-4-aza-4,7β-dimethyl-16β-(4-methylphenoxy)-5α-androstane; 3-oxo-4-aza-4,7β-dimethyl-16β-(tert-butyloxy)-5α-androstane; 3-oxo-4-aza-4,7β-dimethyl-16β-(3-methyl-1-butyloxy)-5α-androstane; 3-oxo-4-aza-4,7β-dimethyl-16α-(n-propyloxy)-5α-androstane; 3-oxo-4-aza-4,7β-dimethyl-16β-(4-trifluoromethylphenoxy)-5α-androstane; 3-oxo-4-aza-4,7β-dimethyl-16β-(4-trifluoromethoxy-phenoxy)-5α-androstane; 3-oxo-4-aza-4,7β-dimethyl-16β-ethylthio-5α-androstane; 3-oxo-4-aza-4,7β-dimethyl-16β-ethylsulfonyl-5α-androstane; 3-oxo-4-aza-4,7β-dimethyl-16β-(4-methylsulfonylphenoxy)-5α-androstane; 3-oxo-4-aza-4,7β-dimethyl-16β-[4-(4-tolylsulfonylamino)phenoxy]-5α-androstane; 3-oxo-4-aza-4,7β-dimethyl-16β-(3-pyridyloxy)-5α-androstane; 3-oxo-4-aza-4,7β-dimethyl-16β-[(4-phenyl) phenoxy]-5α-androstane; 3-oxo-4-aza-4,7β-dimethyl-16β-(4-fluorophenoxy)-5α-androstane; 3-oxo-4-aza-4,7β-dimethyl-16β-(2-pyrazinyloxy)-5α-androstane; 3-oxo-4-aza-4,7β-dimethyl-16β-[4-(5-oxazolyl) phenoxy]-5α-androstane; 3-oxo-4-aza-4,7β-dimethyl-16β-(2-pyrimidinyloxy)-5α-androstane; 3-oxo-4-aza-4,7β-dimethyl-16β-[4-(1-pyrryl)phenoxy]-5α-androstane; 3-oxo-4-aza-4,7β-dimethyl-16β-(4-amino-phenoxy)-5α-androstane; 3-oxo-4-aza-4,7β-dimethyl-16β-(4-acetylaminophenoxy)-5α-androstane; 3-oxo-4-aza-4,7β-dimethyl-16β-(4-benzoylaminophenoxy)-5α-androstane; 3-oxo-4-aza-4,7β-dimethyl-16β-(4-chlorophenoxy)-5α-androstane; 3-oxo-4-aza-4,7β-dimethyl-16β-(phenoxy)-5α-androstane; 3-oxo-4-aza-4,7β-dimethyl-16β-(2-chlorophenoxy)-5α-androstane; 3-oxo-4-aza-4,7β-dimethyl-16β-(3-chlorophenoxy)-5α-androstane; 3-oxo-4-aza-4,7β-dimethyl-16β-(4-chlorophenoxy)-5α-androst-1-ene; 3-oxo-4-aza-4,7β-dimethyl-16-(4-chlorobenzylidene)-5α-androstane; 3-oxo-4-aza-4,7β-dimethyl-16-benzylidene-5α-androstane; 3-oxo-4-aza-4,7β-dimethyl-16-(4-methylbenzylidene)-5α-androstane; 3-oxo-4-aza-4,7β-dimethyl-16-(4-chlorobenzyl)-5α-androstane; 3-oxo-4-aza-4,7β-dimethyl-16-(4-methylbenzyl)-5α-androstane; 3-oxo-4-aza-4,7β-dimethyl-16-(3-pyridylmethyl)-5α-androstane; 3-oxo-4-aza-4,7β-dimethyl-16α-methanesulfonyl-5α-androstane; 3-oxo-4-aza-4,7β-dimethyl-16β-thiophenoxy-5α-androstane;3-oxo-4-aza-4,7β-dimethyl-16β-(4-chlorothiophenoxy)-5α-androstane;3-oxo-4-aza-4,7β-dimethyl-16β-(4-fluoro-thiophenoxy)-5α-androstane; 3-oxo-4-aza-4,7β-dimethyl-16β-(4-methylthiophenoxy)-5α-androstane; 3-oxo-4-aza-4,7β-dimethyl-16β-(4-methoxythiophenoxy)-5α-androstane; 3-oxo-4-aza-4,7β-dimethyl-16β-phenylsulfinyl-5α-androstane; 3-oxo-4-aza-4,7β-dimethyl-16β-phenylsulfonyl-5α-androstane; 3-oxo-4-aza-4,7β, 16α-trimethyl-16β-(4-trifluoromethylphenoxy)-5α-androstane,3-oxo-4-aza-4,7β, 16α-trimethyl-16β-hydroxy-5α-androstane; and 3-oxo-4-aza-4,7(3,16α-trimethyl-16β-methoxy-5α-androstane.

Yet another embodiment administration of a compound of structural formula IV with a testosterone supplement.

In one class of embodiment of structural formula IV, the α-substituent (dashed lines) of =Z is hydrogen and the β-substituent (wedge) of =Z is e.g. methyl, ethyl, propyl, allyl, carboxymethyl, hydroxy, methoxy, ethoxy, cyclopropyloxy, cyclopentyloxy, acetoxy, fluoro, chloro, bromo, trifluoromethyl, fluoromethyl, chloromethyl, carboxy, N,N-dimethylcarbamate, hydroxymethyl, and the like.

In another class of embodiment, =Z is an alkenyl substituent, =CH-R^{3b}, selected from: =CH₂, =CH-CH₃,and =CH-CH₂CH₃-

In yet another class of the present embodiment, NR^{1b}R ^{2b} represents a heterocycle. In one subclass of this class, -NR^{1b}R^{2b} is selected from: N-piperidinyl, N-morpholinyl, N-piperazinyl, N-(4-methyl)piperazinyl, N-thiomorpholinyl, N-pyrrolidinyl, N-imidazolidinyl and the like.

Particular compounds of structural formula IV, useful in the present invention include: 7β-ethyl-4-methyl-4-aza-cholest-5-en-3-one, 7β-ethyl-4-methyl-4-aza-cholestane-3-one, 7β-ethyl-4-aza-cholest-5-en-3-one, 7β-ethyl-4-aza-5α-cholestan-3-one, 7β-carboxymethyl-4-aza-cholest-5-en-3-one, 7β-carboxymethyl-4-aza-cholestan-3-one, 7β-propyl-4-methyl-4-aza-cholest-5-en-3-one, 7β-propyl-4-methyl-4-aza-5α-cholestan-3-one, 7β-propyl-4-aza-cholest-5-en-3-one, 7β-propyl-4-aza-5α-cholestan-3-one, 7β-methyl-4-aza-cholest-5-en-3-one, 7β-methyl-4-aza-cholestan-3-one, 4,7β-dimethyl-4-aza-cholest-5-en-3-one, 4,7β-dimethyl-4-aza-5α-cholestan-3-one, 4-methyl-4-aza-5α-cholestan-3,7-dione, 7β-acetoxy-4-methyl-4-aza-5α-cholestan-3-one, 4-methyl-4-aza-cholest-5-en-3,7-dione, 7β-hydroxy-4-methyl-4-aza-5α-cholestane-3-one, 7 β-methoxy-4-methyl-4-aza-5α-cholestane-3 -one, 7 β-hydroxymethyl-4-aza-5α-cholestane-3 -one, 7β-bromomethyl-4-aza-5α-cholestane-3-one, 7β-chloromethyl-4-aza-5α-cholestane-3-one, 7β-fluoromethyl-4-aza-5α-cholestane-3-one, 7β-carboxy-4-aza-5α-cholestane-3-one, 7β-trifluoromethyl-4-aza-cholest-5-en-3-one, 7,7-dimethoxy-4-methyl-4-aza-5α-cholestane-3-one, 7β-methoxy-4-methyl-4-aza-cholesta-5-en-3-one, 7β-methoxy-4-methyl-4-aza-cholesta-6-en-3-one, 7β-cyclopropyloxy-4-methyl-4-aza-5α-cholestane-3-one, 7β-cyclopropyloxy-4-methyl-4-aza-cholesta-5,7-dien-3-one, 7β-propylidene-4-methyl-4-aza-5α-cholestane-3-one, 7β-(2-ethyl)spiroethylene-4-methyl-4-aza-5α-cholestane-3-one, and 7β-methyl-4-aza-5α-cholest-1-en-3-one.

In yet another embodiment, the compound is selected from: 17β-(N-tert-butylcarbamoyl)-3-oxo-4-aza-5α-androst-1-en-3-one; N-(2,5-bis-trifluoromethyl-phenyl)-3-oxo-4-aza-4-methyl-5α-androst-1-ene-17β-carboxamide; N-(2-trifluoromethyl-phenyl)-3-oxo-4-aza-4-methyl-5α-androst-1-ene-17β-carboxamide;3-oxo-4-aza-7β-methyl-16β-(4-methylphenoxy)-5α-androst-1-ene; 3-oxo-4-aza-4,7β-dimethyl-16p-(phenoxy)-5α-androstane; 3-oxo-4-aza-4,7p-dimethyl-16p-(4-chlorophenoxy)-5α-androstane; and pharmaceutically acceptable salts thereof.

In one class of this embodiment, the compound is selected from: 17β-( *N*-tert-butylcarbamoyl)-3-oxo-4-aza-5α-androst-1-en-3-one; *N-*(2,5-bis-trifluoromethyl-phenyl)-3-oxo-4-aza-4-methyl-5α-androst-1-ene-17β-carboxamide; N-(2-trifluoromethyl-phenyl)-3-oxo-4-aza-4-methyl-5α-androst-1-ene-17β-carboxamide; 3-oxo-4-aza-7β-methyl-16β-(4-methylphenoxy)-5α-androst-1-ene; and pharmaceutically acceptable salts thereof.

"Parkinson's disease" refers to a chronic progressive nervous disease chiefly of later life that is linked to decreased dopamine production in the substantia nigra. Symptoms include stooped posture, resting tremor, weakness of resting muscles, a shuffling gait, speech impediments, movement difficulties and an eventual slowing of mental processes and dementia.

"Male sexual dysfunction" includes impotence, loss of libido, and erectile dysfunction.

"Erectile dysfunction" is a disorder involving the failure of a male mammal to achieve erection, ejaculation, or both. Symptoms of erectile dysfunction include an inability to achieve or maintain an erection, ejaculatory failure, premature ejaculation, or inability to achieve an orgasm. An increase in erectile dysfunction is often associated with age and is generally caused by a physical disease or as a side-effect of drug treatment.

The term "composition" as used herein is intended to encompass a product comprising the specified ingredients in the specified amounts, as well as any product which results, directly or indirectly, from combination of the specified ingredients in the specified amounts. The composition may be used for separate, sequential or combined administration to the subject.

In one embodiment, the "subject" to be treated by the methods and compositions of the present invention is a man with Alzheimer's disease. In another embodiment, the subject is a male human over 50 years old. In one class of this embodiment, the subject is a male human over 55 years old. In another class, the subject is a male human with hypogonadism having serum total testosterone less than 350 ng/dL (hypogonadism being defined as a serum total testosterone level less than the lower limit of normal for younger men [LLN], and recognizing that the LLN will be dependent on the laboratory performing the serum testosterone assay). In another class, the subject is a male human with hypogonadism having serum total testosterone less than 300 ng/dL. In another class, the subject is a male human with hypogonadism having serum total testosterone less than 317 ng/dL. In another class, the subject is a male human with hypogonadism having serum total testosterone less than 280 ng/dL. In another class, the subject is a male human with hypogonadism having serum free testosterone less than 7.344 ng/dL (0.255 nmol/L) . In another class, the subject is a male human with hypogonadism having serum bioavailable testosterone less than 109.4 ng/dL (3.8 nmol/L). In another embodiment, the subject is a human male with partial androgen deficiency with serum total testosterone less than 400 ng/dL. In another embodiment, the subject is a human male with partial androgen deficiency with serum total testosterone less than 432 ng/dL(15 nmol/L).

In another embodiment, the "subject" to be treated by the methods and compositions of the present invention is a man with Parkinson's disease. In another embodiment, the subject is a male human over 50 years old. In one class, the subject is a male human over 55 years old. In another class, the subject is a male human with hypogonadism having serum total testosterone less than 350 ng/dL. In another class, the subject is a male human with hypogonadism having serum total testosterone less than 300 ng/dL. In another class, the subject is a male human with hypogonadism having serum total testosterone less than 317 ng/dL. In another class, the subject is a male human with hypogonadism having serum total testosterone less than 280 ng/dL. In another class, the subject is a male human with hypogonadism having serum free testosterone less than 7.344 ng/dL (0.255 nmovL). In another class, the subject is a male human with hypogonadism having serum bioavailable testosterone less than 109.4 ng/dL (3.8 nmol/L). In another embodiment, the subject is a human male with partial androgen deficiency with serum total testosterone less than 400 ng/dL. In another embodiment, the subject is a human male with partial androgen deficiency with serum total testosterone less than 432 ng/dL(15 nmol/L).

In one embodiment, the "subject" to be treated by the methods and compositions of the present invention is a man with sexual dysfunction. In another embodiment, the subject is a male human over 50 years old. In one class, the subject is a male human over 55 years old. In another class, the subject is a male human with hypogonadism having serum total testosterone less than 350 ng/dL. In another class, the subject is a male human with hypogonadism having serum total testosterone less than 300 ng/dL. In another class, the subject is a male human with hypogonadism having serum total testosterone less than 317 ng/dL. In another class, the subject is a male human with hypogonadism having serum total testosterone less than 280 ng/dL. In another class, the subject is a male human with hypogonadism having serum free testosterone less than 7.344 ng/dL (0.255 nmol/L). In another class, the subject is a male human with hypogonadism having serum bioavailable testosterone less than 109.4 ng/dL (3.8 nmol/L). In another embodiment, the subject is a human male with partial androgen deficiency with serum total testosterone less than 400 ng/dL. In another embodiment, the subject is a human male with partial androgen deficiency with serum total testosterone less than 432 ng/dL(15 nmol/L).

In another embodiment, the subject is a human male with partial androgen deficiency with serum total testosterone <450 ng/dL. In another class of this embodiment, the subject is a male human having a serum total testosterone level <450 ng/dL, as measured by conventional means. In another class of this embodiment, the subject is a male human having a serum total testosterone level <432 ng/dL. In one class of this embodiment, the subject is a male human having a serum testosterone level ≤400 ng/dL. In another class of this embodiment, the subject is a male human having a serum total testosterone level <350 ng/dL. In another class of this embodiment, the subject is a male human having a serum total testosterone level <300 ng/dL. In another class of this embodiment, the subject is a male human having a serum total testosterone level <280 ng/dL

Further, in another embodiment, the male subject has a serum total testosterone level of <200 ng/dL.

In another embodiment, it is provided that subject does not have benign prostatic hyperplasia. In one aspect of this embodiment, it is provided that the subject treated with a compound of structural formula I and a testosterone supplement does not have benign prostatic hyperplasia. In another aspect of this embodiment, it is provided that the subject treated with finasteride or dutasteride and a testosterone supplement does not have benign prostatic hyperplasia.

In another embodiment, it is provided that subject does not have male pattern baldness or androgenic alopecia. In one aspect of this embodiment, it is provided that the subject treated with a compound of structural formula I and a testosterone supplement does not have male pattern baldness or androgenic alopecia. In another aspect of this embodiment, it is provided that the subject treated with finasteride or dutasteride and a testosterone supplement does not have male pattern baldness or androgenic alopecia. In another aspect of this embodiment, it is provided that the subject treated with finasteride and a testosterone supplement does not have male pattern baldness or androgenic alopecia.

In addition to safe improvement in Alzheimer's disease symptoms, Parkinson's disease symptoms and/or erectile/sexual function, the method may also be accompanied by decreased abdominal circumference, decreased fasting serum glucose and insulin levels, reduced hypercholesterolemia, reduced hypertriglyceridemia, increased HDL-C, decreased blood pressure, increased lean body/ muscle mass, decreased total fat mass, decreased C-reactive protein levels, decreased cortisol levels, decreased leptin levels, decreased need for insulin/glucose-regulating agents, increased bone mineral density/bone mass, decreased risk of developing type 2 diabetes, and decreased risk of developing atherosclerosis and associated complications.

The method of the present invention solves the problem of safely elevating testosterone levels in aging men with Alzheimer's disease, Parkinson's disease, and/or erectile/sexual dysfunction, particularly those with low or low-normal testosterone levels by using a well-tolerated, pharmacologic therapy that does not elevate dihydrotestosterone levels. Prior to the present invention, there were no pharmacologic methods of treatment that could safely correct the low/low-normal testosterone levels in these men without also significantly increasing dihydrotestosterone levels.

The term "effective amount" means the amount of 5α-reductase inhibitor that will elicit the biological or medical response of a tissue, system, animal or human that is being sought by the researcher, veterinarian, medical doctor or other clinician. In one embodiment, an effective amount of the compound of structural formula I, II, III, or IV is the amount that reduces serum dihydrotestosterone levels by about 30% or more. If more than one compound of structural formula I, II, III or IV is administered, the total serum DHT lowering is about 60% or more. In one class of the invention, the reduction in serum DHT is about 30%. In another class of the invention, the reduction in serum DHT is more than 60%. In yet another class of the invention, the reduction in serum DHT is more than 90%.

Generally, the daily dosage of the 5α-reductase inhibitor of structural formula I, II, III or IV may be varied over a wide range from 0.01 to 500 mg per adult human per day. In a preferred embodiment, the 5α-reductase inhibitor is administered at a dose of 1.0 to 100 mg per day. In another preferred embodiment, the 5α-reductase inhibitor is administered at a dose of 0.5 to 10 mg per day. For oral administration, the compositions are preferably provided in the form of tablets containing 0.01, 0.05, 0.1, 0.5, 1.0, 2.5, 5.0, 10.0, 15.0, 25.0, 50.0 and 100 milligrams of active ingredient for the symptomatic adjustment of the dosage to the subject to be treated. An effective amount of the drug is ordinarily supplied at a dosage level of from about 0.0002 mg/kg to about 50 mg/kg of body weight per day. The range is more particularly from about 0.001 to 7 mg/kg of body weight per day.

The dose may be administered in a single daily dose or the total daily dosage may be administered in divided doses of two, three or four times daily. Furthermore, when administered via intranasal routes, transdermal routes, by rectal suppositories, or through a continual intravenous solution, the dosage administration will, of course, be continuous rather than intermittent throughout the dosage regimen.

The formulation of the testosterone supplement should provide a dose of testosterone adequate to maintain the male subject's serum total testosterone level within approximately 500 to 600 ng/dL range, based on measures of serum total testosterone. The amount of the testosterone or testosterone derivative present in the composition depends on the patient's starting serum total testosterone and the mode of administration. For oral administration, the compositions are preferably provided in the form of tablets containing 0.01, 0.05, 0.1, 0.5, 1.0, 2.5, 5.0, 10.0, 15.0, 25.0, 50.0 and 100 milligrams of active ingredient for the symptomatic adjustment of the dosage to the subject to be treated. An effective amount of the drug is ordinarily supplied at a dosage level of from about 0.0002 mg/kg to about 50 mg/kg of body weight per day. The range is more particularly from about 0.001 to 7 mg/kg of body weight per day.

In particular, testosterone and testosterone derivative delivered by intramuscular injections may be provided in injections of 50 to 750 mg every 2 to 4 weeks. In one embodiment, testosterone and testosterone derivatives are provided by intramuscular injections of 100 to 500 mg every 2 to 4 weeks. In one class of this embodiment, testosterone and testosterone derivatives are provided by intramuscular injections of 200 to 250 mg every 2 to 4 weeks.

Testosterone and testosterone derivatives may be provided in gel or cream forms in doses of 20 to 200 mg per day. In one embodiment, testosterone and testosterone derivatives are provided in a gel at doses of 50 to 100 mg/day, particularly 50 mg/day, 75 mg/day and 100 mg/day.

Transdermal patches used to deliver testosterone and testosterone derivatives of 1 to 10 mg per day, particularly, 4 to 6 mg/day.

Testosterone and testosterone derivatives may also be provided by means of a buccal gel at a dose of 10mg/day to 100 mg/day. In one embodiment, the dose of testosterone or testosterone derivative buccal gel is 40 to 80 mg/day. In one class of this embodiment, the dose of testosterone or testosterone derivative buccal gel is 60 mg/day.

Formulations of the 5α-reductase inhibitors and the testosterone supplement employed in the present method for medical use comprise the 5α-reductase inhibitor and testosterone supplement together with an acceptable carrier thereof, for separate, sequential or simultaneous administration. The carrier must be pharmaceutically acceptable in the sense of being compatible with the other ingredients of the formulation and not deleterious to the recipient subject of the formulation.

According to the methods of the present invention, for the treatment of sexual dysfunction and erectile dysfunction, the 5α-reductase inhibitor and the testosterone supplement may be administered as the sole active agents or together with another active agent useful in treating sexual dysfunction or erectile dysfunction, such PDE V inhibitors such as sildenafil (VIAGRA), vardenafil (LEVITRA), tadalafil (CIALIS), avanafil, DA159, dasanatafil, SK350; AGE (advanced glycation end-product) breaker such as alagebrium chloride; alpha 1 blocker such as phentolamine mesylate (VASOMAX, ROGITINE); alpha 1A antagonists such as HMP 12; alpha 2 antagonists such as moxisylyte (ERECNOS), yohimbe; dopamine agonists such as apomorphine, NBI69733; dopamine D4 agonists such as ABT724 and AT670; guanylate cyclase stimulants such as BAY632521; melanocortin agonists such as PT141; oxytocin agonists; FR229934; SCH444877, ATB901, JNJ10258859, prostaglandin agonists such as alprostadil (MUSE, ALPROX-TD, VIRIDAL DUO), radical scavengers such as OX008; rotamase inhibitors such as GPI1485; aviptadil; nitroglycerine; GPCR agonists such as R873; a selective androgen receptor modulator (SARM); or with another 5alpha-reductase inhibitor, particularly, another 5α-reductase inhibitor of structural formulae I, II, III or IV. The 5alpha-reductase compound, testosterone supplement and other agent useful for treating sexual dysfunction or erectile dysfunction may be administered separately, sequentially or in a combined preparation.

According to the methods of the present invention, for the treatment of Alzheimer's disease, the 5α-reductase inhibitor and the testosterone supplement may be administered as the sole active agents or together with another active agent useful in treating Alzheimer's disease, such as: tacrine, rivastigmine, galantamine, memantine, antioxidants (vitamins E and C, selenium), Ginkgo biloba, short or medium acting benzodiazepines, cholinergic enhancing agents (donepezil, leuprolide acetate), and nonsteroidal antiinflammatory drugs or with another 5alpha-reductase inhibitor, particularly, another 5α-reductase inhibitor of structural formulae I, II, III or IV. The 5alpha-reductase compound, testosterone supplement and other agent useful for treating Alzheimer's disease may be administered separately, sequentially or in a combined preparation.

According to the methods of the present invention, for the treatment of Parkinson's disease, the 5α-reductase inhibitor and the testosterone supplement may be administered as the sole active agents or together with another active agent useful in treating Parkinson's disease, such as: levodopa/carbidopa, levodopa/benserazide, dopamine receptor agonists (e.g., ropinirole, apomorphine, selegiline, entacapone, bromocryptine, carbergoline, lysuride, pergolide, antimuscarinic drugs (eg., orphenadrine, bezhexol, benztropine and procyclidine), ethopropazine, trihexphenidyl, antidepressants (amitryptaline, doxepine, imipramine, nortriptyline, propanolol), antihistamines (diphenhydramine, orphenadrine), and amantadine, or with another 5alpha-reductase inhibitor, particularly, another 5α-reductase inhibitor of structural formulae I, II, III or IV. The 5alpha-reductase compound, testosterone supplement and other agent useful for treating Parkinson's disease may be administered separately, sequentially or in a combined preparation.

The present invention, therefore, further provides a pharmaceutical formulation comprising a 5α-reductase inhibitor and a testosterone supplement together with a pharmaceutically acceptable carrier thereof, for separate, sequential or simultaneous administration.

The formulations include those suitable for oral, rectal, topical or parenteral (including subcutaneous, intramuscular and intravenous administration). Preferred are those suitable for oral administration.

The formulations may be presented in a unit dosage form and may be prepared by any of the methods known in the art of pharmacy. All methods include the step of bringing the active compound in association with a carrier which constitutes one or more ingredients. In general, the formulations are prepared by uniformly and intimately bringing the active compound in association with a liquid carrier, a waxy solid carrier or a finely divided solid carrier, and then, if needed, shaping the product into the desired dosage form.

Formulations suitable for oral administration may be presented as discrete units such as capsules, cachets, tablets or lozenges, each containing a predetermined amount of the active compound; as a powder or granules; or a suspension or solution in an aqueous liquid or non-aqueous liquid, e.g., a syrup, an elixir, or an emulsion, as well-known in the pharmaceutical arts.

Formulations for rectal administration may be presented as a suppository with a conventional carrier, i.e., a base that is nontoxic and nonirritating to mucous membranes, compatible with the 5α-reductase inhibitors, and is stable in storage and does not bind or interfere with the release of the compound. Suitable bases include: cocoa butter (theobroma oil), polyethylene glycols (such as carbowax and polyglycols), glycol-surfactant combinations, polyoxyl 40 stearate, polyoxyethylene sorbitan fatty acid esters (such as Tween, Myrj, and Arlacel), glycerinated gelatin, and hydrogenated vegetable oils. When glycerinated gelatin suppositories are used, a preservative such as methylparaben or propylparaben may be employed.

Topical preparations containing the active drug component can be admixed with a variety of carrier materials well known in the art, such as, e.g., alcohols, aloe vera gel, allantoin, glycerine, vitamin A and E oils, mineral oil, PPG2 myristyl propionate, and the like, to form, e.g., alcoholic solutions, topical cleansers, cleansing creams, skin gels, skin lotions, and shampoos in cream or gel formulations.

The compounds can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

Compounds may also be delivered by the use of monoclonal antibodies as individual carriers to which the compound molecules are coupled. The compounds of the present invention may also be coupled with soluble polymers as targetable drug carriers. Such polymers can include polyvinylpyrrolidone, pyran copolymer, polyhydroxypropylmethacrylamidephenol, polyhydroxy-ethylaspartamidephenol, or polyethylene-oxide polylysine substituted with palmitoyl residues. Furthermore, the compounds of the present invention may be coupled to a class of biodegradable polymers useful in achieving controlled release of a drug, for example, polylactic acid, polyepsilon caprolactone, polyhydroxy butyric acid, polyorthoesters, polyacetals, polydihydropyrans, polycyanoacrylates and cross-linked or amphipathic block copolymers of hydrogels.

Formulations suitable for parenteral administration include formulations that comprise a sterile aqueous preparation of the active compound that is preferably isotonic with the blood of the recipient. Such formulations suitably comprise a solution or suspension of a compound that is isotonic with the blood of the recipient subject. Such formulations may contain distilled water, 5% dextrose in distilled water or saline and the active compound. Often it is useful to employ a pharmaceutically and pharmacologically acceptable acid addition salt of the active compound that has appropriate solubility for the solvents employed. Useful salts include the hydrochloride isothionate and methanesulfonate salts. Useful formulations also comprise concentrated solutions or solids comprising the active compound which on dilution with an appropriate solvent give a solution suitable for parenteral administration.

The compounds of the present invention may be coupled to a class of biodegradable polymers useful in achieving controlled release of a drug, for example, polylactic acid, polyepsilon caprolactone, polyhydroxy butyric acid, polyorthoesters, polyacetals, polydihydro-pyrans, polycyanoacrylates and cross-linked or amphipathic block copolymers of hydrogels.

The following examples are not intended to be limitations on the scope of the instant invention in any way, and they should not be so construed. Furthermore, examples are not to be construed as forming the only methods and compositions that are considered as the invention. Those skilled in the art will readily understand that known variations of the conditions, processes, methods and compositions of the following preparative procedures can be used.

### EXAMPLE 1

### Preparation of Human Prostatic and Scalp 5α-Reductases

Samples of human tissue were pulverized using a freezer mill and homogenized in 40 mM potassium phosphate, pH 6.5, 5 mM magnesium sulfate, 25 mM potassium chloride, 1 mM phenylmethyl-sulfonyl fluoride, 1 mM dithiothreitol (DTT) containing 0.25 M sucrose using a Potter-Elvehjem homogenizer. A crude nuclear pellet was prepared by centrifugation of the homogenate at 1,500 x g for 15 min. The crude nuclear pellet was washed two times and resuspended in two volumes of buffer. Glycerol was added to the resuspended pellet to a final concentration of 20%. The enzyme suspension was frozen in aliquots at - 80°C. The prostatic and scalp reductases were stable for at least 4 months when stored under these conditions.

### 5α-Reductase Assay

The reaction mixture for the type 1 5α-reductase contained 40 mM potassium phosphate, pH 6.5, 5 mM [7-³H]-testosterone, 1 mM dithiothreitol and 500 µM NADPH in a final volume of 100 µL.The reaction mixture for the type 2 5α-reductase contained 40 mM sodium citrate, pH 5.5, 0.3 mM [7-³H]-testosterone, 1 mM dithiothreitol and 500 µM NADPH in a final volume of 100 µL. Typically, the assay was initiated by the addition of 50-100 µg prostatic homogenate or 75-200 µg scalp homogenate and incubated at 37°C. After 10-50 min the reaction was quenched by extraction with 250 µL of a mixture of 70% cyclohexane: 30% ethyl acetate containing 10 µg each DHT and T. The aqueous and organic layers were separated by centrifugation at 14,000 rpm in an Eppendorf microfuge. The organic layer was subjected to normal phase HPLC (10 cm WHATMAN PARTISIL 5 silica column equilibrated in 1 mL/min 70% cyclohexane: 30% ethyl acetate; retention times: DHT, 6.8-7.2 min; androstanediol, 7.6-8.0 min; T, 9.1-9.7 min). The HPLC system consisted of a WATERS Model 680 Gradient System equipped with a Hitachi Model 655α Autosampler, Applied Biosystems Model 757 variable UV detector, and a Radiomatic Model A120 radioactivity analyzer. The conversion of T to DHT was monitored using the radioactivity flow detector by mixing the HPLC effluent with one volume of Flo Scint 1 (Radiomatic). Under the conditions described, the production of DHT was linear for at least 25 min. The only steroids observed with the human prostate and scalp preparations were T, DHT and androstanediol.

### Inhibition Studies

Compounds were dissolved in 100% ethanol. The compound to be tested was pre-incubated with the enzyme (either 5α-reductase type 1 or 2) prior to initiation by addition of substrate testosterone. IC₅₀ values represent the concentration of inhibitor required to decrease enzyme conversion of testosterone to dihydrotestosterone by 50% of the control. IC₅₀ values were determined using a 6 point titration where the concentration of the inhibitor was varied from 0.1 to 1000 nM. Representative compounds of this invention were tested in the above described assay for 5α-reductase type 1 and type 2 inhibition.

A compound referred to herein as a 5α-reductase 2 inhibitor is a compound that shows inhibition of the 5α-reductase 2 isozyme in the above-described assay, having an IC₅₀ value of about or under 100 nM.

The compounds are tested in the above-described assay for 5α-reductase type 1 and type 2 inhibition, and were found to have IC₅₀ values under about 100 nM for inhibition of the type 1 isozyme. Compounds found to have IC₅₀ values of under about 50 nM for inhibition of the type 1 isozyme are called type 1 inhibitors.

The compounds called "dual inhibitors" were inhibitors of both 5α-reductase type 1 and 5α-reductase type 2 as defined above.

### EXAMPLE 2

### Rat Ex Copula Assay

Sexually mature male Caesarian Derived Sprague Dawley (CD) rats (over 60 days old) are used with the suspensory ligament surgically removed to prevent retraction of the penis back into the penile sheath during the ex copula evaluations. Animals receive food and water *αd lib* and are kept on a normal light/dark cycle. Studies are conducted during the light cycle.

1) Conditioning to Supine Restraint for Ex Copula Reflex Tests. This conditioning takes ~ 4 days. Day 1, the animals are placed in a darkened restrainer and left for 15 - 30 minutes. Day 2, the animals are restrained in a supine position in the restrainer for 15 - 30 minutes. Day 3, the animals are restrained in the supine position with the penile sheath retracted for 15 - 30 minutes. Day 4, the animals are restrained in the supine position with the penile sheath retracted until penile responses are observed. Some animals require additional days of conditioning before they are completely acclimated to the procedures; non-responders are removed from further evaluation. After any handling or evaluation animals are given a treat to ensure positive reinforcement.

2) Ex Copula Reflex Tests. Rats are gently restrained in a supine position with their anterior torso placed inside a cylinder of adequate size to allow for normal head and paw grooming. For a 400-500 gram rat, the diameter of the cylinder is approximately 8 cm. The lower torso and hind limbs are restrained with a non-adhesive material (vetrap). An additional piece of vetrap with a hole in it, through which the glans penis will be passed, is fastened over the animal to maintain the preputial sheath in a retracted position. Penile responses will be observed, typically termed *ex copula* genital reflex tests. Typically, a series of penile erections will occur spontaneously within a few minutes after sheath retraction. The types of normal reflexogenic erectile responses include elongation, engorgement, cup and flip. An elongation is classified as an extension of the penile body. Engorgement is a dilation of the glans penis. A cup is defined as an intense erection where the distal margin of the glans penis momentarily flares open to form a cup. A flip is a dorsiflexion of the penile body.

Baseline and or vehicle evaluations are conducted to determine how and if an animal will respond. Some animals have a long duration until the first response while others are non-responders altogether. During this baseline evaluation latency to first response, number and type of responses are recorded. The testing time frame is 15 minutes after the first response.

After a minimum of 1 day between evaluations, these same animals are administered the test compound at 20 mg/kg and evaluated for penile reflexes. All evaluations are videotaped and scored later. Data are collected and analyzed using paired 2 tailed t-tests to compared baseline and/ or vehicle evaluations to drug treated evaluations for individual animals. Groups of a minimum of 4 animals are utilized to reduce variability.

Positive reference controls are included in each study to assure the validity of the study. Animals can be dosed by a number of routes of administration depending on the nature of the study to be performed. The routes of administration includes intravenous (IV), intraperitoneal (IP), subcutaneous (SC) and intracerebral ventricular (ICV).

### EXAMPLE 3

### In vivo experiment

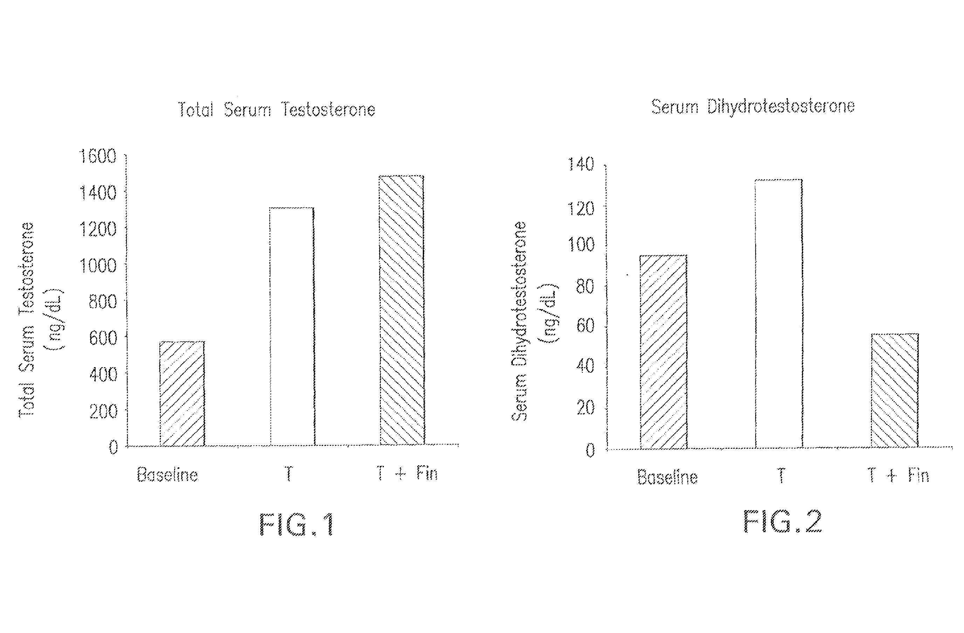

The effects of a topical cream containing testosterone (T) alone versus a topical cream containing T plus finasteride were examined in a 43 year old male patient. On Day 1, the patient's total serum T and dihydrotestosterone (DHT) levels were measured at 11:30 a.m. On Day 2, at 6:30 a.m., the patient applied to his chest a topical cream (10 grams) containing testosterone 1% (100 mg), and the patient's T and DHT levels were again measured at 11:30 a.m. that morning. The patient did not wash off the cream until the morning of Day 3. On Day 6, at 6:30 a.m., the patient applied to his chest a topical cream (10 grams; same cream base as used for T alone) containing testosterone 1% (100 mg) plus finasteride 50 mg, and the patient's T and DHT levels were again measured at 11:30 a.m. that morning. The patient did not wash off the cream until the morning of Day 7. As shown in FIGURES 1 and 2, application of the topical cream containing T alone led to an increase in total serum T and DHT. Relative to the hormone levels obtained at baseline and those obtained following application of the topical cream containing T alone, application of the topical cream containing T plus finasteride led to a further increase in serum T and a reduction in serum DHT (the DHT level was decreased below the original baseline level).

While the invention has been described and illustrated with reference to certain particular embodiments thereof, those skilled in the art will appreciate that various changes, modifications and substitutions can be made therein without departing from the spirit and scope of the invention. For example, effective dosages other than the particular dosages as set forth herein above may be applicable as a consequence of variations in the responsiveness of the subject being treated for any of the indications for the compounds of the invention indicated above. Likewise, the specific pharmacological responses observed may vary according to and depending upon the particular active compound selected or whether there are present pharmaceutical carriers, as well as the type of formulation and mode of administration employed, and such expected variations or differences in the results are contemplated in accordance with the objects and practices of the present invention. It is intended, therefore, that the invention be defined by the scope of the claims which follow and that such claims be interpreted as broadly as is reasonable.

## Claims

1. The combination of a testosterone supplement together with a 5alpha-reductase inhibitor for use in a method of treating a human male subject with erectile dysfunction, wherein the 5alpha-reductase inhibitor is selected from a compound of structural formulae I, II, III and IV, or a pharmaceutically acceptable salt thereof
wherein, structural formula I is: 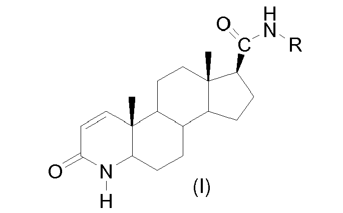 wherein R is selected from:
(a) C₁₋₁₀ alkyl, unsubstituted or substituted with one to three halogen substituents, and
(b) phenyl, unsubstituted or substituted with one to three substituents independently selected from halogen, methyl, and trifluoromethyl;
wherein structural formula II is: 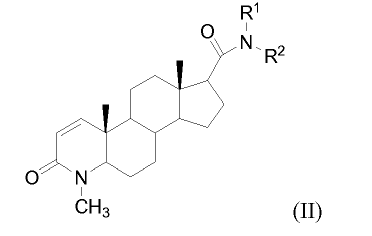 wherein:
R¹ is selected from
(a) H, and
(b) C₁-₆ alkyl;
R² is selected from:
(a) diarylmethyl, either unsubstituted or substituted on one or both of the aryl rings with one to three substituents independently selected from:
(1) halo (F, Cl, Br, I),
(2) C₁₋₂ alkyl,
(3) trifluoromethyl,
(4) nitro,
(5) hydroxy,
(6) cyano,
(7) phenyl,
(8) C₁₋₂ alkyloxy,
(9) heteroaryl,
(10) S(O)ₙR³, wherein n is selected from 0, 1, and 2, and
(11) alkyoxy;
(b) phenyl substituted with one to three substituents independently selected from:
(1) halo (F, Cl, Br, I),
(2) C₁₋₂ alkyl;
(3) trifluoromethyl,
(4) nitro,
(5) hydroxy,
(6) cyano,
(7) phenyl,
(8) C₁₋₂ alkyloxy,
(9) heteroaryl,
(10) S(O)ₙR³, wherein n is selected from 0, 1, and 2, and
(11) alkyoxy;
(c) heteroaryl, either unsubstituted or substituted with one to three substituents independently selected from:
(1) halo (F, Cl, Br, I),
(2) C₁₋₂ alkyl;
(3) trifluoromethyl,
(4) nitro,
(5) hydroxy,
(6) cyano,
(7) amino,
(8) C₁₋₂ alkyloxy,
(9) phenyl, and
(10) heteroaryl;
R³ is selected from:
(a) C₁₋₄ alkyl,
(b) phenyl, and
(c) heteroaryl;
wherein structural formula III is: 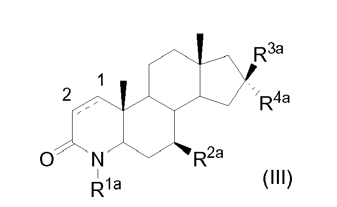 wherein:
the C1-C2 carbon-carbon bond may be a single bond, or a double bond as indicated by the dashed line; R^{1a} is selected from the group consisting of hydrogen and methyl;
R^{2a} is selected from the group consisting of hydrogen and C₁-₁₀ alkyl;
one of R^{3a} and R^{4a} is selected from the group consisting of hydrogen and methyl, and the other is selected from the group consisting of:
(a) amino;
(b) cyano;
(c) fluoro,
(d) methyl;
(e) OH;
(f) -C(O)NR_{b}R_{c}, where R_{b} and R_{c} are independently H, C₁₋₆ alkyl, aryl, or arylC_{1- 6}alkyl; wherein the alkyl moiety can be substituted with 1-3 of: halo; C₁₋₄alkoxy; or trifluoromethyl; and the aryl moiety can be substituted with 1-3 of: halo; C₁₋₄alkyl; C₁₋₄ alkoxy; or trifluoromethyl;
(g) C₁₋₁₀ alkyl-X-;
(h) C₂₋₁₀ alkenyl-X-; wherein the C₁-₁₀ alkyl in (g) and C₂₋₁₀alkenyl in (h) can be unsubstituted or substituted with one to three of:
*(i)* halo; hydroxy; cyano; nitro; mono-, di- or trihalomethyl; oxo; hydroxysulfonyl; carboxy;
*(ii)* hydroxyC₁₋₆alkyl; C₁₋₆alkyloxy; C₁₋₆ alkylthio; C₁₋₆alkylsulfonyl; C_{1- 6} alkyloxycarbonyl; in which the C₁₋₆ alkyl moiety can be further substituted with 1-3 of: halo; C₁₋₄ alkoxy; or trifluoromethyl;
*(iii)* arylthio; aryl; aryloxy; arylsulfonyl; aryloxycarbonyl; in which the aryl moiety can be further substituted with 1-3 of: halo; C₁₋₄ alkyl; C₁₋₄ alkoxy; or trifluoromethyl;
(iv) -C(O)NR_{b}R_{c}; -N(R_{b})-C(O)-R_{c}; -NR_{b}R_{c}; where R_{b} and R_{c} are defined above;
(i) aryl-X-;
(j) heteroaryl-X-, wherein heteroaryl is a 5, 6 or 7 membered heteroaromatic ring containing at least one member selected from the group consisting of: one ring oxygen atom, one ring sulfur atom, 1-4 ring nitrogen atoms , or combinations thereof; in which the heteroaromatic ring can also be fused with one benzo or heteroaromatic ring; wherein the aryl in (i) and heteroaryl in (j) can be unsubstituted or substituted with one to three of:
(v) halo; hydroxy; cyano; nitro; mono-, di- or trihalomethyl; mono-, di- or trihalomethoxy; C₂-₆ alkenyl; C₃₋₆ cycloalkyl; formyl; hydrosulfonyl; carboxy; ureido;
(vi) C₁₋₆ alkyl; hydroxy C₁₋₆ alkyl; C₁₋₆ alkyloxy; C₁₋₆ alkyloxy C₁₋₆alkyl; C₁₋₆ alkylcarbonyl; C₁₋₆ alkylsulfonyl; C₁₋₆ alkylthio; C₁₋₆ alkylsulflnyl; C₁₋₆ alkylsulfonamido; C₁₋₆ alkylarylsulfonamido; C₁₋₆ alkyloxy-carbonyl; C₁₋₆ alkyloxycarbonyl C₁₋₆alkyl; R_{b}R_{C}N-C(O)-C_{1 6}alkyl; C₁₋₆ alkanoylamino C₁₋₆ alkyl; aroylamino C₁₋₆ alkyl; wherein the C₁₋₆ alkyl moiety can be substituted with 1-3 of: halo; C₁₋₄alkoxy; or trifluoromethyl;
*(vii)* aryl; aryloxy; arylcarbonyl; arylthio; arylsulfonyl; arylsulfinyl; arylsulfonamido; aryloxycarbonyl; wherein the aryl moiety can be substituted with 1-3 of: halo; C₁₋₄alkyl; C₁₋₄alkoxy; or trifluoromethyl;
*(viii)* -C(O)NR_{b}R_{c}; -O-C(O)-NR_{b}R_{c}; -N(Rb)-C(O)-R_{c}; -NR_{b}R_{c}; Rb-C(O)-N(R_{c})-; where R_{b} and R_{c} are defined in (f) above; and -N(R_{b})-C(O)-OR_{g} wherein Rg is C₁₋₆alkyl or aryl, in which the alkyl moiety can be substituted with 1-3 of: halo; C₁₋₄alkoxy; or trifluoromethyl, and the aryl moiety can be substituted with 1-3 of: halo; C₁₋₄alkyl; C₁₋₄ alkoxy, or trifluoromethyl; -N(R_{b})-C(O) NR_{c}R_{d}, wherein R_{d} is selected from H, C₁₋₆ alkyl, and aryl; in which said C₁₋₆alkyl and aryl can be substituted as described above in (f) for R_{b} and R_{c};
(ix) a heterocyclic group, which is a 5, 6 or 7 membered ring, containing at least one member selected from the group consisting of: one ring oxygen atom, one ring sulfur atom, 1-4 ring nitrogen atoms, or combinations thereof; in which the heterocyclic ring can be aromatic, unsaturated, or saturated, wherein the heterocyclic ring can be fused with a benzo ring, and wherein said heterocyclic ring can be substituted with one to three substituents, as defined above for *v*), *vi*)*, vii*) and *viii*)*,* excluding *ix*) a heterocyclic group; and
(k) R^{3a} and R^{4a} taken together can be carbonyl oxygen;
(l) R^{3a} and R^{4a} taken together can be =CH-Rg wherein Rg is defined in *viii);* and wherein:
X is selected from the group consisting of:
- O-; -S(O)ₙ-; -C(O)-; -CH(Rₑ)-; -C(O)-O-*; -C(O)-N(Rₑ)-*;
- N(Rₑ)-C(O)-O-*; -O-C(O)-N(Rₑ)-*; -N(Rₑ)C(O)-N(Rₑ)-;
- O-CH(Rₑ)-*; -N(Re)-; wherein Rₑ is H, C₁₋₃ alkyl, aryl, aryl- C₁₋₃ alkyl, or unsubstituted or substituted heteroaryl, as defined above in (j);
wherein the asterisk (*) denotes the bond which is attached to
the 16-position in Structure III; and n is zero, 1 or 2;
and wherein each alkyl and alkenyl moiety can be unsubstituted or substituted with one or more, and preferably 1 to three, of:
(i) halo; hydroxy; cyano; nitro; mono-, di- or trihalomethyl; oxo; hydroxysulfonyl; carboxy;
*(ii)* hydroxyC ₁₋₆alkyl; C₁₋₆alkyloxy; C₁₋₆alkylthio; C ₁₋₆alkylsulfonyl; C₁₋₆ alkyloxycarbonyl; in which the C₁₋₆ alkyl moiety can be further substituted with 1-3 of: halo; C₁₋₄ alkoxy; or trifluoromethyl;
*(iii)* arylthio; aryl; aryloxy; arylsulfonyl; aryloxycarbonyl; in which the aryl moiety can be further substituted with 1-3 of: halo; C₁₋₄ alkyl; C₁₋₄ alkoxy; or trifluoromethyl; and
(iv) -C(O)NR_{b}R_{c}; -N(R_{b})-C(O)-R_{c}; -NR_{b}R_{c}; where R_{b} and R_{c} are defined above;
and halo is F, Cl, Br or I;
wherein structural formula IV is: 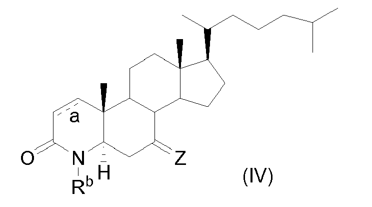 wherein:
Rb is selected from hydrogen and methyl;
the dashed line " - - -" a represents a single bond or a double bond;
=Z is selected from:
(1) oxo,
(2) α-hydrogen and a β-substituent selected from:
(a) C₁-C₄ alkyl,
(b) C₂-C₄ alkenyl,
(c) CH₂COOH,
(d) -OH,
(e) -COOH,
(f) -COO(C₁₋C₄ alkyl),
(g) -OCONR^{1b}R^{2b} wherein R^{1b} and R^{2b} independently are selected from:
(i) H,
(ii) C₁₋C₄ alkyl,
(iii) phenyl, and
(iv) benzyl, or
R^{1b} and R^{2b} together with the nitrogen atom to which they are attached represent a 5-6 membered saturated heterocycle, optionally containing one other heteratom selected from -O-, -S- and -N(R')- wherein R' is -H or methyl;
(h) C₁-C₄ alkoxy,
(i) C₃-C₆ cycloalkoxy,
(j) -OC(O)-C₁₋₄ alkyl,
(k) halo,
(l) hydroxy-C₁₋C₂ alkyl,
(m) halo-C₁₋C₂ alkyl,
(n) -CF3 and
(O) C₃-C₆ cycloalkyl;
(3) =CHR3b; wherein R3b is selected from -H and C₁-C₄ alkyl.

2. The combination according to Claim 1 wherein the human male subject has serum testosterone levels less than 432 ng/dL.

3. The combination according to Claim 1, wherein the testosterone supplement is selected from: testosterone precursors, prodrugs, analogs, and androgen receptor agonists.

4. The method according to Claim 3, wherein the testosterone supplement is selected from:
dehydroepiandrosterone, androstenedione, testosterone enanthate, testoterone propionate, testosterone cypionate, methyltestosterone, fluoxy mesterone, 17-α methyl testosterone, balasterone, clostebol, formebolone, nadrolone, oxymesterone, quinbolone, and salts and esters thereof.

5. The combination according to Claim 1 wherein the 5alpha-reductase inhibitor is administered in an amount that reduces serum dihydrotestosterone levels by about 30% or more when administered to the male subject.

6. The combination according to Claim 1 wherein the 5alpha-reductase compound is selected from:
17β-(N-tert-butylcarbamoyl)-3-oxo-4-aza-5α-androst-1-en-3-one;
N-(2,5-bis-trifluoromethyl-phenyl)-3-oxo-4-aza-4-methyl-5α-androst-1-ene-17β-carboxamide;
N-(diphenylmethyl)-4-methyl-3-oxo-4-aza-5α-androst-1-ene-17β-carboxamide;
N-(diphenylmethyl)-N-methyl-4-methyl-3-oxo-4-aza-5α-androst-1-ene-17β-carboxamide;
N-(2-methylphenyl)-3-oxo-4-aza-4-methyl-5α-androst-1 -ene-17β-carboxamide;
N-(2-methoxyphenyl)-3-oxo-4-aza-4-methyl-5α-androst-1-ene-17β-carboxamide;
N-(2-chlorophenyl)-3-oxo-4-aza-4-methyl-5α-androst 1-ene-17β-carboxamide;
N-(4-chlorophenyl)-3-oxo-4-aza-4-methyl-5α-androst-1-ene-17β-carboxamide;
N-(2-fluorophenyl)-3 -oxo-4-aza-4-methyl-5α-androst-1-ene-17β-carboxamide;
N-(2-trifluoromethyl-phenyl)-3-oxo-4-aza-4-methyl-5α-androst-1-ene-17β-carboxamide;
N-(2,5-bistrifluoromethyl-phenyl)-3-oxo-4-aza-4-methyl-5α-androst-1-ene-17β-carboxamide;
N-(2-biphenyl)-3 -oxo-4-aza-4-methyl-5α-androst-1-ene-17β-carboxamide;
N-(4-biphenyl)-3-oxo-4-aza-4-methyl-5α-androst-1-ene-17β-carboxamide;
N-(4-pyridyl)-3-oxo-4-methyl-4-aza-5α-androst-1-ene-17β-carboxamide;
N-(3-pyridyl)-3-oxo-4-methyl-4-aza-5α-androst-1-ene-17β-carboxamide;
N-(pyrazinyl)-3-oxo-4-methyl-4-aza-5α-androst-1-ene-17β-carboxamide;
N-(3-pyrazoyl)-3-oxo-4-methyl-4-aza-5α-androst-1-ene-17β-carboxamide;
N-(2-thiazolyl)-3-oxo-4-aza-4-methyl-5α-androst-1-ene-17β-carboxamide;
4-aza-4,7β-dimethyl-5α-androstane-3,16-dione;
4-aza-4-methyl-5α-androstan-3,16-dione;
3-oxo-4-aza-4-methyl-16β-hydroxy-5α-androstane;
3-oxo-4-aza-4-methyl-16β-(benzylaminocarbonyloxy)-5α-androstane;
3-oxo-4-aza-4-methyl-16β-benzoylamino-5α-androstane;
3-oxo-4-aza-4-methyl-16β-methoxy-5α-androstane;
3-oxo-4-aza-4-methyl-16β-allyloxy-5α-androstane;
3-oxo-4-aza-4-methyl-16β-(n-propyloxy)-5α-androstane;
3-oxo-4-aza-4-methyl-16α-hydroxy-5α-androstane;
3-oxo-4-aza-4-methyl-16β-(phenoxy)-5α-androstane;
3 -oxo-4-aza-7β-methyl-16β-(phenoxy)-5α-androst-1-ene;
3-oxo-4-aza-4-methyl-16α-methoxy-5α-androstane;
3-oxo-4-aza-4-methyl-16β-(4-chlorophenoxy)-5α-androstane;
3-oxo-4-aza-7β-methyl-16β-(4-chlorophenoxy)-5α-androst-1-ene;
3 -oxo-4-aza-7β-methyl-16β-(4-chlorophenoxy)-5α-androstane;
3-oxo-4-aza-7β-methyl-16β-(3-chloro-4-methylphenoxy)-5α-androstane;
3-oxo-4-aza-7β-methyl-16β-(4-methylphenoxy)-5α-androstane;
3-oxo-4-aza-7β-methyl-16β-(4-methylphenoxy)-5α-androst-1-ene;
3-oxo-4-aza-7β-methyl-16β-[4-(1-pyrrolyl)phenoxy]-5α-androst-1-ene;
3-oxo-4-aza-4,7β-dimethyl-16β**-**hydroxy-5α-androstane;
3-oxo-4-aza-4,7β**-**dimethyl-16β-methoxy-5α-androstane;
3-oxo-4-aza-4,7β-dimethyl-16β-allyloxy-5α-androstane;
3-oxo-4-aza-4,7β-dimethyl-16β-(3,3-dimethylallyloxy)-5α-androstane;
3-oxo-4-aza-4,7β-dimethyl-16β-(n-propyloxy)-5α-androstane;
3-oxo-4-aza-4,7β-dimethyl-16β-(iso-pentoxy)-5α-androstane;
3 -oxo-4-aza-4,16α-dimethyl-16β-hydroxy-5α-androstane;
3-oxo-4-aza-4,7β**-**dimethyl-16β-ethyloxy-5α-androstane;
3-oxo-4-aza-4,7β-dimethyl-16β-benzyloxy-5α-androstane;
3-oxo-4-aza-4,7β**-**dimethyl-16α-hydroxy-5α-androstane;
3-oxo-4-aza-4,7β-dimethyl-16β-methylthio-5α-androstane;
3-oxo-4-aza-4,7β-dimethyl-16β-(n-propylthio)-5α-androstane;
3-oxo-4-aza-4,7β-dimethyl-16β-fluoro-5α-androstane;
3-oxo-4-aza-4,7β-dimethyl-16β-cyano-5α-androstane;
3-oxo-4-aza-4,7β**-**dimethyl-16β-(1-hexyl)-5α-androstane;
3-oxo-4-aza-4,7β-dimethyl-16β-(n-propyl)-5α-androstane;
3-oxo-4-aza-4,7β-dimethyl-16β-benzyl-5α-androstane;
3-oxo-4-aza-4,7β-dimethyl-16β-(4-chlorobenzyl)-5α-androstane;
3-oxo-4-aza-4,16α-dimethyl-16β-methoxy-5α-androstane;
3-oxo-4-aza-4,7β-dimethyl-16β-(4-cyanophenoxy)-5α-androstane;
3-oxo-4-aza-4,7β-dimethyl-16β-(3-cyanophenoxy)-5α-androstane;
3-oxo-4-aza-4,7β-dimethyl-16β-(4-mtrophenoxy)-5α-androstane;
3-oxo-4-aza-4,7β-dimethyl-16β-(1-naphthyloxy)-5α-androstane;
3-oxo-4-aza-4,7β-dimethyl-16β-(3 -chloro-4-methylphenoxy)-5α-androstane;
3-oxo-4-aza-4,7β-dimethyl-16β-(4-methylphenoxy)-5α-androstane;
3-oxo-4-aza-4,7β-dimethyl-16β-(tert-butyloxy)-5α-androstane;
3-oxo-4-aza-4,7β-dimethyl-16β-(3-methyl-1-butyloxy)-5α-androstane;
3-oxo-4-aza-4,7β-dimethyl-16α-(n-propyloxy)-5α-androstane;
3-oxo-4-aza-4,7β-dimethyl-16β-(4-trifluoromethylphenoxy)-5α-androstane;
3-oxo-4-aza-4,7β-dimethyl-16β-(4-trifluoromethoxyphenoxy)-5α-androstane;
3-oxo-4-aza-4,7β-dimethyl-16β-ethylthio-5α-androstane;
3-oxo-4-aza-4,7β-dimethyl-16β-ethylsulfonyl-5α-androstane;
3-oxo-4-aza-4,7β-dimethyl-16β-(4-methylsulfonylphenoxy)-5α-androstane;
3-oxo-4-aza-4,7β-dimethyl-16β-[4-(4-tolylsulfbnylamino)phenoxy]-5α-androstane;
3-oxo-4-aza-4,7β-dimethyl-16β-(3-pyridyloxy)-5α-androstane;
3-oxo-4-aza-4,7β-dimethyl-16β-[(4-phenyl)phenoxy)-5α-androstane;
3-oxo-4-aza-4,7β-dimethyl-16β-(4-fluorophenoxy)-5α-androstane;
3-oxo-4-aza-4,7β-dimethyl-16β-(2-pyrazinyloxy)-5α-androstane;
3-oxo-4-aza-4,7β-dimethyl-16β-[4-(5-oxazolyl)phenoxy]-5α-androstane;
3-oxo-4-aza-4,7β-dimethyl-16β-(2-pynmidmyloxy)-5α-androstane;
3-oxo-4-aza-4,7β-dimethyl-16β-[4-(1-pyrryl)phenoxy]-5α-androstane;
3-oxo-4-aza-4,7β-dimethyl-16β-(4-aminophenoxy)-5α-androstane;
3-oxo-4-aza-4,7β-dimethyl-16β-(4-acetylaminophenoxy)-5α-androstane;
3-oxo-4-aza-4,7β-dimethyl-16β-(4-benzoylaminophenoxy)-5α-androstane;
3-oxo-4-aza-4,7β-dimethyl-16β-(4-chlorophenoxy)-5α-androstane;
3-oxo-4-aza-4,7β-dimethyl-16β-(phenoxy)-5α-androstane;
3-oxo-4-aza-4,7β-dimethyl-16β-(2-chlorophenoxy)-5α-androstane;
3-oxo-4-aza-4,7β-dimethyl-16β-(3-chlorophenoxy)-5α-androstane;
3-oxo-4-aza-4,7β-dimethyl-16β-(4-chlorophenoxy)-5α-androst-1-ene;
3-oxo-4-aza-4,7β-imethyl-16-(4-chlorobenzylidene)-5α-androstane;
3-oxo-4-aza-4,7β-dimethyl-16-benzylidene-5α-androstane;
3-oxo-4-aza-4,7β-dimethyl-16-(4-methylbenzylidene)-5α-androstane;
3-oxo-4-aza-4,7β-dimethyl-16-(4-chlorobenzyl)-5α-androstane;
3-oxo-4-aza-4,7β-dimethyl-16-(4-methylbenzyl)-5α-androstane;
3-oxo-4-aza-4,7β-dimethyl-16-β-pyridylmethyl)-5α-androstane;
3-oxo-4-aza-4,7β-dimethyl-16α-methanesulfonyl-5α-androstane;
3-oxo-4-aza-4,7β-dimethyl-16β-thiophenoxy-5α-androstane;
3-oxo-4-aza-4,7β-dimethyl-16β-(4-chlorothiophenoxy)-5α-androstane;
3-oxo-4-aza-4,7β-dimethyl-16β-(4-fluorothiophenoxy)-5α-androstane;
3-oxo-4-aza-4,7β-dimethyl-16β-(4-methylthiophenoxy)-5α-androstane;
3-oxo-4-aza-4,7β-dimethyl-16β-(4-methoxythiophenoxy)-5α-androstane;
3-oxo-4-aza-4,7β-dimethyl-16β-phenylsulfinyl-5α-androstane;
3-oxo-4-aza-4,7β-dimethyl-16β-phenylsulfonyl-5α-androstane;
3-oxo-4-aza-4,7β,16α-trimethyl-16β-(4-trifluoromethylphenoxy)-5α-androstane,
3-oxo-4-aza-4,7β,16α-trimethyl-16β-hydroxy-5α-androstane;
3-oxo-4-aza-4,7β,16α-trimethyl-16β-methoxy-5α-androstane;
7β-ethyl-4-methyl-4-aza-cholest-5-en-3-one;
7 β-ethyl-4-methyl-4-aza-cholestane-3 -one;
7 β-ethyl-4-aza-cholest -5-en-3-one;
7β-ethyl-4-aza-5α-cholestan-3-one;
7 β-carboxymethyl-4-aza-cholest-5-en-3-one;
7 β-carboxymethyl-4-aza-cholestan-3 -one;
7 β-propyl-4-methyl-4-aza-cholest-5-en-3-one;
7β-propyl-4-methyl-4-aza-5α-cholestan-3-one;
7 β-propyl-4-aza-cholest-5-en-3-one;
7β-propyl-4-aza-5α-cholestan-3-one;
7 β-methyl-4-aza-cholest-5-en-3-one;
7 β-methyl-4-aza-cholestan-3 -one;
4,7 7β-dimethyl-4-aza-cholest-5-en-3-one;
4,7β-dimethyl-4-aza-5α-cholestan-3-one;
4-methyl-4-aza-5α-cholestan-3,7-dione;
7β-acetoxy-4-methyl-4-aza-5α-cholestan-3-one;
4-methyl-4-aza-cholest-5-en-3,7-dione;
7β-hydroxy-4-methyl-4-aza-5α-cholestane-3-one;
7β-methoxy-4-methyl-4-aza-5α-cholestane-3-one;
7β-hydroxymethyl-4-aza-5α-cholestane-3-one;
7β-bromomethyl-4-aza-5α-cholestane-3-one;
7β-chloromethyl-4-aza-5α-cholestane-3-one;
7β-fluoromethyl-4-aza-5α-cholestane-3-one;
7β-carboxy-4-aza-5α-cholestane-3-one;
7β-trifluoromethyl-4-aza-cholest-5-en-3-one;
7,7-dimethoxy-4-methyl-4-aza-5α-cholestane-3-one;
7β-methoxy-4-methyl-4-aza-cholesta-5-en-3-one;
7β-methoxy-4-methyl-4-aza-cholesta-6-en-3 -one;
7β-cyclopropyloxy-4-methyl-4-aza-5α-cholestane-3 -one;
7β-cyclopropyloxy-4-methyl-4-aza-cholesta-5,7-dien-3-one;
7β-propylidene-4-methyl-4-aza-5α-cholestane-3-one;
7β-(2-ethyl)spiroethylene-4- methyl-4-aza-5α-cholestane-3-one; and
7β-methyl-4-aza-5α-cholest-1-en-3-one;
or a pharmaceutically acceptable salt thereof.

7. The combination according to Claim 6, wherein the compound is selected from:
17β-(N-tert-butylcarbamoyl)-3-oxo-4-aza-5α-androst-1-en-3-one,
N-(2,5-bis-trifluoromethyl-phenyl)-3-oxo-4-aza-4-methyl-5α-androst-1-ene-17β-carboxamide;
N-(2-trifluoromethyl-phenyl)-3-oxo-4-aza-4-methyl-5α-androst-1-ene-17β-carboxamide;
3-oxo-4-aza-7β-methyl-16β-(4-methylphenoxy)-5α-androst-I-ene;
3-oxo-4-aza-4,7β-dimethyl-16β-(phenoxy)-5α-androstane;
3-oxo-4-aza-4,7β-dimethyl-16β-(4-chlorophenoxy)-5α-androstane;
or a pharmaceutically acceptable salt thereof.

8. The combination according to Claim 1, wherein the method comprises administering a compound of structural formula I and a compound of structural formula III.

9. The combination according to Claim 7, wherein the compound is selected from finasteride and 3-oxo-4-aza-7β-methyl-16β-(4-methylphenoxy)-5α-androst-1-ene.

10. The combination according to Claim 1, wherein the method comprises administering 3-oxo-4-aza-7β-methyl-16β-(4-methylphenoxy)-5α-androst-1-ene as the inhibitor of 5 alpha reductase.

11. A pharmaceutical composition comprising:
a 5alpha reductase inhibitor compound selected from a compound according to Claim 3 of structural formulae I, II, III and IV;
a testosterone supplement;
a compound selected from: sildenafil, vardenafil, tadalafil, avanafil, DA159, dasanatafil, SK350,
alagebrium chloride, phentolamine mesylate, HMP 12, moxisylyte, yohimbe. spomorphine, NBI69733, ABT724, AT670, BAY632521, PT141; FR229934; SCH444877, ATB901, JNJ10258859 alprostadil, OX008, GPI1485, aviptadil, nitroglycerine, and R873;
and a pharmaceutically acceptable carrier.
